(19) Europäisches Patentamt
European Patent Office
Office européen des brevets

(11) Veröffentlichungsnummer: **0 205 775**
**A2**

(12) # EUROPÄISCHE PATENTANMELDUNG

(21) Anmeldenummer: **86104157.2**

(22) Anmeldetag: **26.03.86**

(51) Int. Cl.[4]: **A61K 31/35** , A61K 31/365 , A61K 31/36 , A61K 35/78

(30) Priorität: **29.03.85 DE 3511609**

(43) Veröffentlichungstag der Anmeldung: **30.12.86 Patentblatt 86/52**

(84) Benannte Vertragsstaaten: **AT BE CH DE FR GB IT LI LU NL SE**

(71) Anmelder: **Moon, Chang-Kiu Dr.**
**Hyundae-villa 101 Ho 96-1, Daechi-Dong**
**Kangnam-Gu Seoul(KR)**

(72) Erfinder: **Moon, Chang-Kiu Dr.**
**Hyundae-villa 101 Ho 96-1, Daechi-Dong**
**Kangnam-Gu Seoul(KR)**

(74) Vertreter: **Brehm, Hans-Peter, Dr. Dipl.-Chem. et al**
**Patentanwälte Tischer, Kern & Brehm**
**Albert-Rosshaupter-Strasse 65**
**D-8000 München 70(DE)**

(54) **Arzneimittel, enthaltend Caesalpinia-Komponenten.**

(57) Ein neues Arzneimittel enthält als Wirkstoff einen aus Pflanzenmaterial der zur Gattung Caesalpinia gehörenden Pflanzen extrahierbaren Extrakt, eine oder mehrere Komponente(n) dieses Extraktes und/oder eine oder mehrere in diesem Extrakt vorkommende oder davon abgeleitete, jedoch synthetisch hergestellte Substanz(en). Bei diesen Substanzen handelt es sich insbesondere um Benz(b)-indeno(2.1-d)pyran-Derivate, einschließlich Brasilin, Brasilein, Hämatoxylin und/oder Hämatein. Das Arzneimittel hat insbesondere eine vorbeugende und/oder heilende Wirkung auf mikrozirkulatorische Störungen, eine antidiabetische Wirkung, eine Blutzucker-senkende Wirkung, eine die Plättchenaggregation hemmende Wirkung, eine die Blutviskosität senkende Wirkung , eine die Erythrozyten-Verformbarkeit steigernde Wirkung, eine normalisierende Wirkung auf die lysosomalen Enzyme und/oder eine den Prostaglandin-Stoffwechsel beeinflußende, hier insbesondere selektiv Thromboxan $A_2$ hemmende Wirkung.

EP 0 205 775 A2

# Arzneimittel, dessen Herstellung und Verwendung

Die Erfindung betrifft ein neues Arzneimittel. Dieses Arzneimittel ist insbesondere für die Anwendung am Menschen bestimmt.

In erster Linie betrifft die Erfindung ein gegen Diabetes mellitus wirksames Arzneimittel.

Ferner vermag das erfindungsgemäße Arzneimittel auch in Verbindung oder als Folge von Diabetes mellitus auftretende Symptome und Schäden zu lindern. In dieser Hinsicht betrifft die Erfindung ein Arzneimittel, das eine den Prostaglandin-Stoffwechsel beeinflussende Wirkung, insbesondere eine die Bildung von Thromboxan $A_2$ hemmende Wirkung hat, und auf diesem Wege die Plättchenaggregation hemmt. Ferner normalisiert das erfindungsgemäße Arzneimittel die Tätigkeit der lysosomalen Enzyme, senkt die Blutviskosität und hat eine die Verformbarkeit der Ery throzyten steigernde Wirkung. Aufgrund dieser Wirkungen kann das erfindungsgemäße Arzneimittel beispielsweise zur Behandlung von Arteriosklerose, der Makro-und Mikroangiopathie sowie zur Förderung der Mikrozirkulation eingesetzt werden.

Weiterhin betrifft die Erfindung ein Verfahren zur Herstellung des Arzneimittels.

Schließlich betrifft die Erfindung die Verwendung bestimmter Substanzen und Substanzgemische als Wirkstoffe in Arzneimitteln, insbesondere in einem Arzneimittel, das eine vorbeugende und/oder heilende Wirkung auf mikrozirkulatorische Störungen hat, das als Antidiabetikum einsetzbar ist, das eine Blutzucker-senkende Wirkung hat, das eine Plättchenaggregation-hemmende Wirkung hat, das eine Blutviskosität-senkende Wirkung hat, das eine die Erythrozyten-Verformbarkeit-steigernde Wirkung hat, das eine die Aktivität der Lysosomalen Enzyme normalisierende Wirkung hat, das eine den Prostaglandin-Stoffwechsel beeinflußende Wirkung hat und/oder das eine selektiv Thromboxan $A_2$ hemmende Wirkung hat.

Die Erfindung wird nachstehend insbesondere im Hinblick auf die Behandlung von Diabetes mellitus erläutert, ohne daß damit eine Einschränkung verbunden sein soll. Vielmehr ergeben sich die Anwendungsmöglichkeiten aus der nachstehenden Beschreibung und den Ansprüchen.

Diabetes mellitus oder "Zuckerkrankheit" ist eine im allgemeinen fortschreitende Störung des Kohlehydrat-sowie des Fett-und Eiweißstoffwechsels infolge Insulinmangels oder verminderter Insulinwirksamkeit und kommt insbesondere in den höheren Altersstufen vor (Typ II-Diabetes). Typische Anzeichen sind ein zu hoher Glukosespiegel im Blut und/oder eine verzögerte Abnahme und Normalisierung des Glukosespiegels nach Nahrungsaufnahme. Ein typischer Test zur Früherkennung ist der Glukose-Toleranztest (GTT).

Nach Erkrankung gehören zu typischen und in regelmäßigen Abständen durchgeführte Untersuchungen die Bestimmung des Nüchternblutzuckers und der post-prandialen Zuckerwerte.

Herkömmliche, zur Behandlung von Diabetes mellitus eingesetzte Arzneimittel, sog. hypoglykämische Mittel, haben eine Blutzucker-senkende Wirkung, die relativ rasch eintritt, beispielsweise innerhalb eines Tages nach oraler Verabreichung. Setzt ein chronischer Diabetiker die regelmäßige Einnahme solcher hypoglykämischer Mittel ab, so kommt es bald zu pathologisch hohen Blutzuckerwerten über ungefähr 120 mg/dl.

Die Aufgabe der vorliegenden Erfindung besteht darin, ein neues Arzneimittel zur Behandlung von Diabetes mellitus bereitzustellen.

Ein weiteres Ziel der Erfindung besteht darin, ein neues Arzneimittel bereitzustellen, das bei Diabetikern eine Verringerung der ständigen Zuführung hypoglykämischer Mittel erlaubt.

Ein weiteres Ziel der Erfindung besteht darin, ein neues Arzneimittel bereitzustellen, das bei Diabetikern vom Typ II eine Verringerung der ständigen Zuführung hypoglykämischer Mittel erlaubt.

Ein weiteres Ziel der Erfindung besteht darin, ein neues Arzneimittel bereitzustellen, das nach einer gewissen Zeitspanne der Verabreichung an Diabetiker vom Typ II die Absetzung hypoglykämischer Mittel erlaubt.

Ein weiteres Ziel der Erfindung besteht darin, ein neues Arzneimittel bereitzustellen, das eine der Mikroangiopathie vorbeugende und/oder eine die Mikrozirkulation fördernde Wirkung hat, insbesondere über eine Hemmung der Plättchenaggregation, über eine Steigerung der Erythrozyten-Verformbarkeit, über eine Senkung der Blutviskosi tät und/oder über eine Normalisierung der Lysosomalen Enzyme.

Schließlich besteht ein weiteres Ziel der Erfindung darin, ein Verfahren zur Herstellung dieses neuen Arzneimittels anzugeben.

Die erfindungsgemäße Lösung dieser Aufgabe und Ziele ist ein Arzneimittel der aus einem der Ansprüche 1, 7, 8, 9 oder 10 ersichtlichen Zusammensetzung.

Vorteilhafte Ausgestaltungen und Weiterbildungen der Erfindung ergeben sich aus den Unteransprüchen.

Ein erster Gesichtspunkt der Erfindung betrifft ein Arzneimittel, das als Wirkstoff einen aus Pflanzenmaterial der zur Gattung Caesalpinia gehörenden Pflanzen extrahierbaren Extrakt, eine oder mehrere Komponente(n) dieses Extraktes und/oder eine oder mehrere in diesem Extrakt vorkommende(n), oder davon abgeleitete(n), jedoch synthetisch hergestellte Substanz(en) enthält.

Zu den in diesem Extrakt vorkommenden Substanzen gehören insbesondere Brasilin, Brasilein, Hämatoxylin und/oder Hämatein.

Das Arzneimittel ist vorzugsweise als oral oder rektal applizierbares Arzneimittel oder als injizierbares Mittel konfektioniert, beispielsweise als Kapsel mit einem Extraktgehalt von etwa 100 bis 700 mg, insbesondere mit 250 mg für oral oder rektal applizierbare Mittel. Soweit andere Angaben fehlen, wird nachstehend unter "das Präparat" eine Kapsel verstanden, die 250 mg trockenen, pulverförmigen Extrakt enthält, deren physiologisch verträgliches Wandmaterial sich im Magen/Darmtrakt auflöst und dort den Extrakt freisetzt. Ein für eine Injektion konfektioniertes Mittel enthält vorzugsweise ca. 60 bis 120 mg Wirkstoff.

Dieser Extrakt weist überraschende physiologische Eigenschaften auf.

Wird das Präparat 3x täglich an Diabetiker verabreicht -anfänglich gemeinsam mit einem hypoglykämischen Mittel, dessen Dosis jedoch allmählich verringert und das schließlich ganz abgesetzt wird -so beobachtet man zumeist nach etwa 14 Tagen nach der ersten Verabreichung eine geringe bis gute Absenkung des Blutzuckerspiegels. Bei stetiger Verabreichung des Präparates in gleichbleibender Dosis (3 $\times$ 250 mg Extrakt pro 60 kg Lebendgewicht), tritt von Woche zu Woche eine langsame, aber stetige Abnahme des Blutzuckerspiegels auf. Etwa 2 bis 12 Monate nach Aufnahme der Behandlung erreicht der Nüchternblutzucker übliche Werte unter 120 mg/dl.

Auch der post-prandiale Glukosewert erreicht ca. 3 bis 24 Monate nach Beginn der Behandlung Normalwerte.

Auch der Glukose-Toleranztest ergibt mit fortschreitender Behandlung langsam aber stetig immer bessere Werte.

Nachdem alle drei Tests -ohne jegliche Zufuhr hypoglykämischer Mittel -Normalwerte erreicht haben, wird die Verabreichung des Präparates noch 1 bis 2 Monate fortgesetzt und dann beendet. Ohne weitere Zufuhr des Präparates und/oder hypoglykämischer Mittel zeigen die Patienten daraufhin ein normales, beschwerdefreies Verhalten, sofern besondere Belastungen (schweres Essen, übermäßiger Alkoholgenuß und dergleichen) vermieden werden.

Generell wirkt das erfindungsgemäße Arzneimittel bei Diabetikern vom Typ II. Bei Insulinabhängigen Diabetikern (Typ I) tritt eine geringere Wirkung auf. Bei solchen Patienten erlaubt die zusätzliche Verabreichung des erfindungsgemäßen Arzneimittels häufig eine Verringerung der sonst notwendigen Insulingaben. Bei einigen wenigen Patienten tritt nur eine geringe Wirkung auf; auch diese Pa tienten berichten jedoch über eine Verbesserung des gesamten Lebensgefühls.

Im Rahmen der vorliegenden Erfindung ist eine Reihe von Untersuchungen durchgeführt worden, um Anhaltspunkte für den Wirkungsmechanismus des erfindungsgemäßen Arzneimittels zu gewinnen. Für diese Untersuchungen wurde als Wirkstoff entweder der mit Wasser aus Holz von Caesalpinia Sappan extrahierbare, getrocknete, pulverförmige Gesamtextrakt, aus diesem Extrakt isoliertes Brasilin und/oder synthetisch hergestelltes Brasilin eingesetzt.

Diese Untersuchungen bestätigten eine Steigerung der peripheren Aktivität von Insulin; beispielsweise konnte an peripheren Erythrozyten eine Erhöhung der Anzahl der Insulinrezeptoren nachgewiesen werden.

Aufgrund dieser Ergebnisse scheint das erfindungsgemäße Arzneimittel eine bessere Ausnutzung des körpereigenen sowie des zugeführten Insulins zu ermöglichen. Hierin ist offensichtlich die Ursache dafür zu sehen, daß nach Verabreichung des erfindungsgemäßen Arzneimittels an Patienten, die auf die Zufuhr von Insulin angewiesen sind, die zur Senkung des Blutzuckerspiegels erforderliche tägliche Insulindosis gesenkt werden kann.

Bei Diabetikern ist bekanntlich der Glukuronsäure-Stoffwechsel aktiviert. Weil die Aktivität der lysosomalen Enzyme (wie etwa $\alpha$-Mannosidase, $\alpha$-und $\beta$-Glukosidase, $\beta$-Galactosidase, $\beta$-Glukuronidase, n-Acetyl-$\beta$-D-Glukosaminidase und dergleichen) nicht normal ist, insbesondere erniedrigt ist, kommt es zu einer Ablagerung von Glykoproteinen an der Basalmembran. Hierin wird eine Ursache gesehen für bekannte Spätschäden von Diabetes mellitus, wie etwa Angiopathie (einschl. Mikro-und Makroangiapathie), Arteriosklerose und dergleichen. Obige Untersuchungen bestätigen eine Besserung dieser Spätschäden nach Verabreichung des erfindungsgemäßen Arzneimittels, was ge gebenenfalls auf eine Hemmung der Plättchenaggregation und/oder auf eine Normalisierung der Aktivität der lysosomalen Enzyme zurückführbar ist, für die Anzeichen gefunden wurden. Diese Wirkung ist wenig abhängig oder unabhängig vom Blutzuckerniveau.

Bekanntlich tritt bei Diabetikern ein erhöhter Thromboxan $A_2$-Spiegel auf, insbesondere aus der Tätigkeit der Thrombozyten. Ein erhöhter Thromboxan $A_2$-Spiegel fördert die Arteriosklerosebildung

und beeinträchtigt die Mikrozirkulation durch Stimulierung der Plättchenaggregation. Herkömmliche Prostaglandinsynthesehemmer, wie etwa Acetylsalicylsäure oder Indomethazin hemmen die Aktivität der Zyklooxygenase , womit zwar die erwünschte Hemmung der Thromboxan-Synthetase erreicht wird, wobei jedoch auch eine unerwünschte Hemmung der Prostazyklin-Synthetase auftritt. Demgegenüber hemmt das erfindungsgemäße Arzneimittel selektiv lediglich die Bildung von Thromboxan $A_2$, ohne die Bildung von Prostazyklin zu hemmen. Damit erlaubt das erfindungsgemäße Arzneimittel eine gezielte Beeinflussung des Prostaglandinstoffwechsels in Richtung einer Hemmung der Thromboxan $A_2$-Bildung. Nachgewiesen wurde diese Wirkung anhand einer Hemmung der Bildung von Malonsäuredialdehyd, ohne daß ein Einfluß auf die Bildung von Prostazyklin festgestellt werden konnte. Bekanntlich wird Malonsäuredialdehyd von dem gleichen Enzymsystem erzeugt, das auch die Bildung von Prostazyklin und Thromboxan $A_2$ veranlaßt.

Weiterhin tritt bei Diabetikern typischerweise eine Verringerung der Erythrozyten-Verformbarkeit und eine Steigerung der Blutviskosität auf. An diabetisch eingestellten Ratten mit diesen Symptomen konnte nach Verabreichung von Gesamtextrakt aus Caesalpina Sappan oder nach Verabreichung von Brasilin eine signifikante Erhöhung der Erythrozyten-Verformbarkeit und eine signifikante Abnahme der Blutviskosität gegenüber der Kontrollgruppe festgestellt werden.

Ein zweiter Gesichtspunkt der Erfindung betrifft ein Verfahren zur Herstellung des erfindungsgemäßen Arzneimittels.

Der Wirkstoff des erfindungsgemäßen Arzneimittels ist ein Extrakt, der aus Pflanzenmaterial der zur Gattung Caesalpini gehörenden Pflanzen extrahierbar ist, eine oder mehrere Komponente(n) des Extraktes und/oder eine oder mehrere in diesem Extrakt vorkommende oder davon abgeleitete, jedoch synthetisch hergestellte Substanzen enthält.

Die Gattung Caesalpinia umfaßt vor allem tropische Pflanzen, insbesondere Bäume, die vor allem in Mittel-und Südamerika und Ostasien vorkommen. Zu einzelnen Vertretern gehören Caesalpinia Sappan (Sappanholz), Caesalpinia Echinata - (Brasilholz), Hämatoxylon Campechianum (Blauholz, Campecheholz), Caesalpinia Coriaria - (Divi-Divi), Caesalpinia Brasiliensi, Hämatoxylon Braziletto und dergleichen. Aus der Literatur ist bekannt, daß zu den Inhaltsstoffen dieser meist intensiv rot oder blau gefärbten Hölzer Brasilin und/oder Hämatoxylin gehören, die in frischem Holz vermutlich glykosidisch gebunden sind. Die Angabe "Pflanzenmaterial, der zur Gattung Caesalpinia gehörenden Pflanzen" soll insbesondere solche Pflanzen einschließen, die Brasilin und/oder Hämatoxylin zu synthetisieren vermögen. Nach Prüfung verschiedener Hölzer der Gattung Caesalpinia ist insbesondere mit Caesalpinia Sappan gearbeitet worden.

Zur Gewinnung des Extraktes kann man von frischem oder trockenem Pflanzenmaterial, vorzugsweise Holz, ausgehen. Besonders bevorzugt wird Kernholz eingesetzt. Das Holz wird zerkleinert, beispielsweise geraspelt, und daraufhin mit einem Lösungsmittel extrahiert. Als Lösungsmittel kommen übliche Lösungsmittel in Betracht, wie beispielsweise Wasser, niedere Alkohole (wie etwa Methanol, Äthanol und Propanol), Diäthyläther, ferner höher siedende Äther (wie etwa Tetrahydrofuran und Dioxan), aliphatische Ketone (wie etwa Aceton oder Methyläthylketon), aliphatische Kohlen wasserstoffe (wie etwa n-Hexan oder Cyclohexan), chlorierte Kohlenwasserstoffe (wie etwa Tetrachlorkohlenstoffe, Chloroform, Dichlormethan), Acetylacetat und dergleichen.

Im Hinblick auf die Löslichkeit wird vorzugsweise mit heißem Lösungsmittel extrahiert; beispielsweise kann die Aufschlämmung der Holzteilchen im Lösungsmittel am Rückfluß gekocht werden.

Im industriellen Maßstab könnte auch mit kaltem Lösungsmittel extrahiert werden, insbesondere wenn eine längere Extraktionsdauer (beispielsweise mehrere Tage) vorgesehen wird. Auch kann die Löslichkeit durch eine Steigerung des pH-Wertes erhöht werden.

Eine Extraktionsdauer von mehreren Stunden ist zweckmäßig. Das einmal extrahierte Holz kann mit frischem Lösungsmittel versetzt und die Extraktion ein oder mehrmals wiederholt werden. Die erhaltenen flüssigen Extrakte werden dann vereinigt. Schließlich wird das Lösungsmittel entfernt und man erhält einen trockenen Rückstand, der zweckmäßigerweise pulverisiert wird.

Besonders gute Ergebnisse werden beim Auskochen mit Wasser erhalten, und dieses Extraktionsverfahren wird vorzugsweise angewandt. 1 Gew.-Teil feingeraspeltes Holz, insbesondere Holz von Caesalpinia-Sappan, wird mit ca. 10 Gew.-Teilen neutralem, entionisiertem Wasser versetzt und bei freiem Luftzutritt 6 bis 8 h lang gekocht. Anschliessend wird das tief braun-rot gefärbte Wasser abgegossen, und die bereits behandelte Holzmasse noch ein-oder zweimal mit der gleichen Wassermenge ausgekocht. Die Extrakte werden vereinigt und das Wasser entfernt, was zweckmäßigerweise durch Verdampfen auf dem Wasserbad oder durch Gefriertrocknen erfolgen kann. Man erhält einen dunkelroten trockenen Rückstand in einer Menge von ca. 2 bis 10% des Holzgewichtes. Im jeweiligen Rückstand (Gesamtextrakt) kann Brasilin und Brasilein und im Gesamtextrakt einiger Pflanzen zusätzlich

Hämatoxylin und Hämatein säulenchromatographisch und/oder mittels Hochdruck-Flüssigkeits-Chromatographie (HPLC) nachgewiesen werden. Der Rückstand (Gesamtextrakt) wird pulverisiert und das anfallende Pulver kann unmittelbar in dieser Form zu einem oral oder rektal applizierbaren oder zu einem injezierbaren Arzneimittel konfektioniert werden. Vorzugsweise werden nach üblichen galenischen Verfahren Kapseln mit einem Pulvergewicht von 250 mg erzeugt.

Fig. 1 zeigt ein IR-Spektrum (KBr-Preßling) im Wellenzahlen-Bereich von 3000 bis 800 cm' des nach dem Auskochen von Sappanholz mit Petroläther erhaltenen dunkelroten Pulvers.

Fig. 2 zeigt ein entsprechendes IR-Spektrum des beim Auskochen von Kernholz von Caesalpinia Sappan mit Wasser erhaltenen trockenen Gesamtextraktes.

Je nach Ausgangsmaterial enthält der Extrakt ca. 1 bis 5 Gew.-% Brasilin und/oder Brasilein. Die chinoide Form des Brasileins entsteht offensichtlich durch Luftoxidation unter den Extraktionsbedingungen. Andere Hölzer, wie insbesondere Hämatoxylon Campechianum liefern eine geringere Menge an Brasilin, dafür einen größeren Anteil an Hämatoxylin und/oder Hämatein. Untersuchungsergebnisse zeigen, daß die Verabreichung von synthetisch hergestelltem Brasilin oder synthetisch hergestelltem Hämatoxylin im wesentlichen die gleichen Wirkungen hervorruft, wie die Verabreichung einer größeren Menge des Gesamtextraktes. Es wird deshalb angenommen, daß es sich bei zumindest einer wirksamen Komponente des Gesamtextraktes um Benz(b)indeno(2.1-d)pyran-Derivate des nachstehenden Strukturtyps (I bzw. II) handelt,

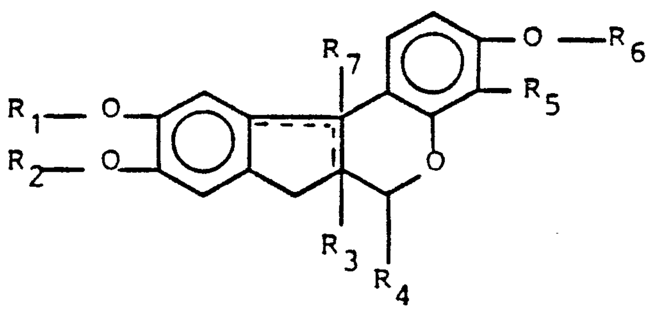

(I)

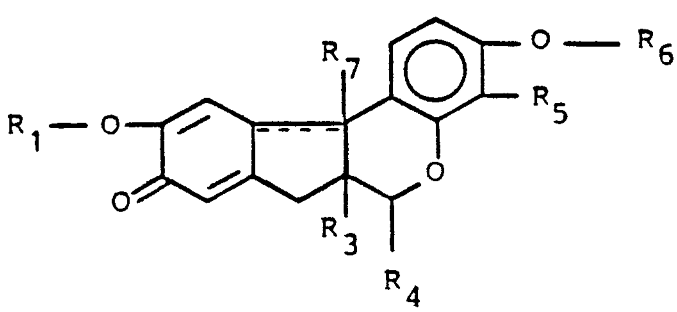

(II)

wobei

$R_1$, $R_2$ und $R_6$ gleich oder verschieden sein können und stehen für einen Wasserstoffrest, einen niederen Alkylrest, vorzugsweise Methylrest, einen Acetylrest, einen Benzylrest, oder $R_1$ und $R_2$ gemeinsam eine Methylengruppe bilden;

$R_3$ und $R_7$ gleich oder verschieden sein können und stehen für einen Wasserstoffrest, Hydroxylrest oder Acetylrest;

$R_4$ für einen Wasserstoffrest oder eine Oxogruppe steht;

$R_5$ für einen Wasserstoffrest, einen Hydroxylrest, einen niederen Alkylrest, vorzugsweise Methylrest, einen Acetylrest oder einen Benzylrest steht; und

- - - - - - - - - - - - für eine Einfachbindung oder für eine Doppelbindung steht.

Zu den mit obiger allgemeiner Strukturformel wiedergegebenen hauptsächlich pharmakologisch aktiven Substanzen gehören insbesondere nachstehende Verbindungen:

1) Brasilin, nämlich

7.11b -Dihydrobenz(b)indeno(2.1-d)pyran-3,6a, 9,10(6H)tetrol

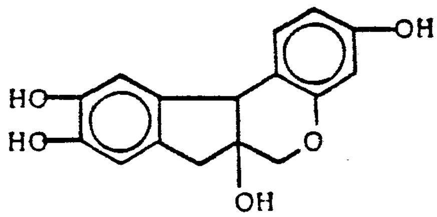

2) Brasilein, nämlich

6a,7-Dihydro-3,6a,10-trihydrobenz(b)indeno(2,1-d)-pyran-9(6H)on

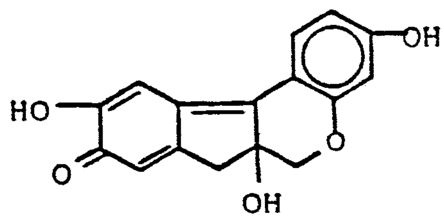

3) Hämatoxylin, nämlich

7.11b-Dihydrobenz(b)indeno(2,1-d)pyran-3,4,6a,9,10(6H)pentol

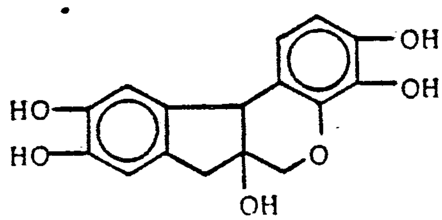

4) Hämatein, nämlich

6a,7-Dihydro-3,4,6a,10-tetrahydroxybenz(b)indeno-(2,1-d)pyran-9(6H)-on

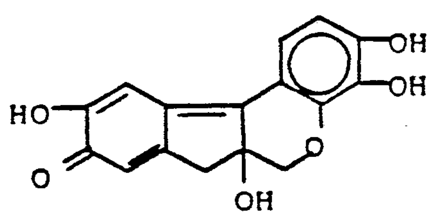

5) O-Triacetylanhydrobrasilin

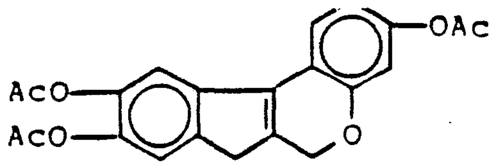

6) O-Trimethoxyindenokumarin

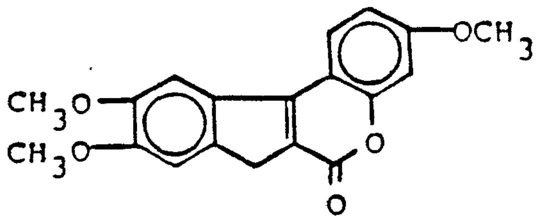

7) O-Trimethylanhydrobrasilan

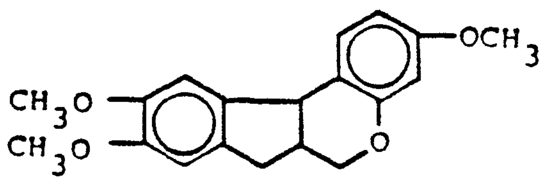

8) O-Triacetyl-cis-brasilan

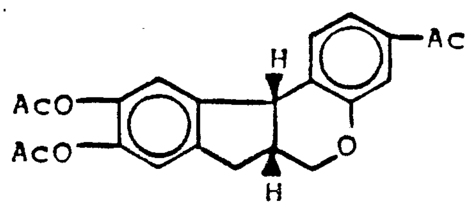

9) Bis(o-triacetyl-cis-brasilan-4-yl)

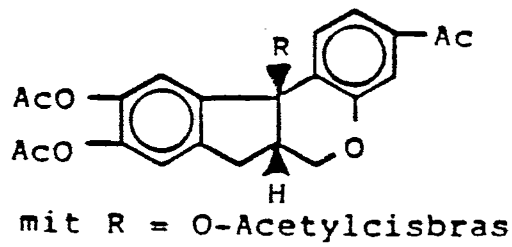

mit R = O-Acetylcisbrasilan-4-yl

10) 7-Hydroxy 5',6'-methylendioxyindeno-(2',3';3,4)kumarin

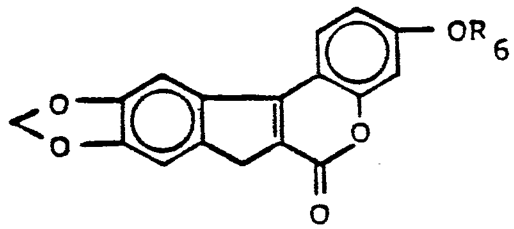

mit $R_6$ = H oder $CH_3$

11) Derivative von Brasilin

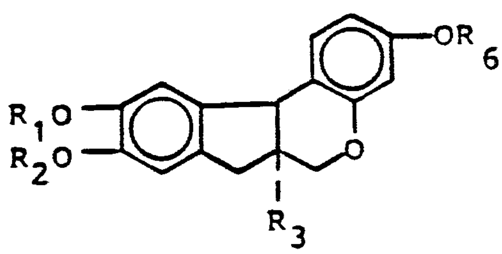

| | | |
|---|---|---|
| Trimethylbrasilin: | $R_1=R_2=R_6=CH_3$ | $R_3$=OH |
| Trimethylacetylbrasilin: | $R_1=R_2=R_6=CH_3$ | $R_3$=oAc |
| Benzylbrasilin: | $R_1=R_2=R_6$=Benzyl | $R_3$=OH |
| Benzylacetylbrasilin: | $R_1=R_2=R_6$=Benzyl | $R_3$=oAc |

12) Desoxytrimethylbrasilin

13) Isobrasilin-Eisen(III)chlorid-trimethyläther

14) Trimethylbrasilon

15) Trimethylbromodeoxybrasilin

Hämatoxylin ($C_{16}H_{14}O_6 \bullet 3H_2O$, MG 302,29+54,05) und Brasilin ($C_{16}H_{14}O_5$, MG 268,29) sind bekannte Substanzen, können im Fachhandel bezogen werden (beispielsweise von FLUKA Feinchemikalien GmbH, Neu-Ulm) und sind insbesondere zum Anfärben von Zellpräparaten verwendet worden. Vereinzelt sind in der wissenschaftlichen Literatur auch physiologische Wirkungen beschrieben worden. So sollen Brasilin und Hämatoxylin entzündungshemmende Wirkung haben (Planta Med. 31, 214-220 (1977). Auch eine antibakterielle Wirkung wurde beschrieben (Rev. Latinoam. Microbiol. 1, 225-232 (1958). Jüngere Ergebnisse haben Auswirkung von Brasilin und Hämatoxylin auf die Lipidperoxidation in den Mitochondrien von Rattenleber (Arch.Pharm.Res. 7, 63-64 (1984) oder eine Plasmalipid-senkende Wirkung auf spontan hypertensive Ratten aufgezeigt (Fette-Seifen-Anstrichmittel, 87, S. 74-76 (1985). Jedoch sind bislang diese Substanzen enthaltende Arzneimittel nicht bekannt geworden.

Ein dritter Gesichtspunkt der Erfindung betrifft die Verwendung bestimmter Substanzen und Substanzgemische als Wirkstoff in einem Arzneimittel. Diese Substanzen und Substanzgemische sind insbesondere brauchbar als Wirkstoff in einem gegen Diabetes mellitus wirksamen Arzneimittel.

Weiterhin sind diese Substanzen und Substanzgemische brauchbar als Wirkstoff in einem Arzneimittel, das eine vorbeugende und/oder heilende Wirkung auf mikrozirkulatorische Störungen hat, das eine Blutzucker-senkende Wirkung hat, das eine Plättchenaggregation hemmende Wirkung hat, das eine Blutviskosität-senkende Wirkung hat, das eine die Erythrozyten-Verformbarkeit steigernde Wirkung hat, das eine die Aktivität der Lysosomalen Enzyme normalisierende Wirkung hat, das eine den Prostaglandin-Stoffwechsel beeinflußende Wirkung hat und/oder das eine selektiv Thromboxan $A_2$ hemmende Wirkung hat.

In diesem Sinne betrifft die Erfindung ein Verfahren zur Behandlung eines Patienten, der an einer der genannten Krankheiten, Beschwerden und/oder Störungen leidet, wobei die Behandlung darin besteht, daß dem Patienten eine wirksame Menge der nachstehend im einzelnen aufgeführten Substanzen und Substanzgemische verabreicht wird.

Zu den in diesem Sinne einsetzbaren Substanzen und Substanzgemischen gehören:

A) Aus Pflanzenmaterial der zur Gattung Caesalpinia gehörenden Pflanzen isolierbare Extrakte, einschl. der aus Holz von Caesalpinia Sappan, von Hämatoxylon Campechanium, von Caesalpinia Echinata, von Caesalpinia Coriaria, von Caesalpinia Brasiliensis und von Hämatoxylon Braziletto isolierbaren Gesamt-Extrakte. Vorzugsweise werden die durch Auskochen mit Wasser gewinnbaren Extrakte eingesetzt. Besonders bevorzugt wird der durch Auskochen von Holz von Caesalpinia Sappan mit Wasser gewinnbare Gesamt-Extrakt verwendet. Ferner können Gemische von Gesamt-Extrakten verwendet werden, die aus verschiedenen Pflanzen der Gattung Caesalpinia isoliert worden sind.

B) Benz(b)indeno(2.1-d)pyran-Derivate mit nachstehenden allgemeinen Strukturformeln (I bzw. II)

(I)

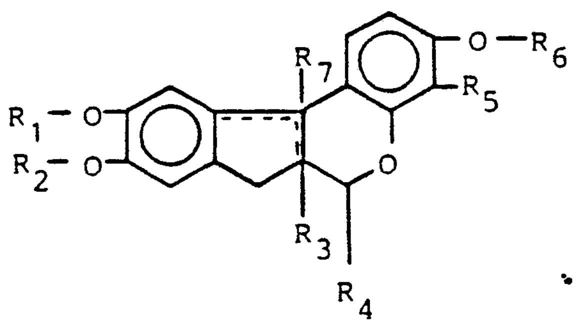

(II)

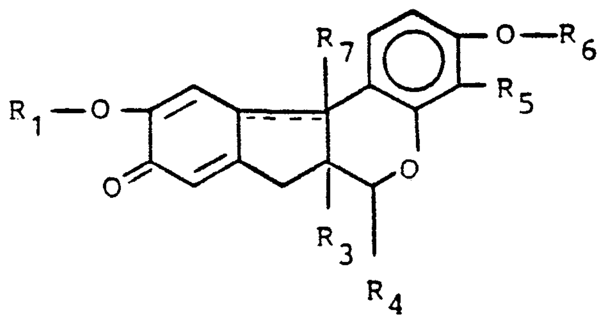

wobei

$R_1$, $R_2$ und $R_6$ gleich oder verschieden sein können und stehen für einen Wasserstoffrest, einen niederen Alkylrest, vorzugsweise Methylrest, einen Acetylrest, einen Benzylrest oder $R_1$ und $R_2$ gemeinsam eine Methylengruppe bilden;

$R_3$ und $R_7$ gleich oder verschieden sein können und stehen für einen Wasserstoffrest, Hydroxylrest oder Acetylrest;

$R_4$ für einen Wasserstoffrest oder eine Oxogruppe steht;

$R_5$ für einen Wasserstoffrest, einen Hydroxylrest, einen niederen Alkylrest, vorzugsweise Methylrest, einen Acetylrest oder einen Benzylrest steht; und

············ für eine Einfachbindung oder für eine Doppelbindung steht.

Zu geeigneten Benz(b)indeno(2.1-d)pyran-Derivaten gehören insbesondere die oben im einzelnen bezeichneten, hauptsächlich pharmakologisch wirksamen Verbindungen - (1) bis (15) einschließlich Brasilin, Brasilein, Hämatoxylin und Hämatein, wobei Brasilin besonders bevorzugt wird.

C) Der oben unter (A) bezeichnete Extrakt, der mit einem oder mehreren, vorstehend unter (B) aufgeführten Benz(b)indeno(2.1-d)pyran-Derivat(en) einschließlich Brasilin, Brasilein, Hämatoxylin und/oder Hämatein, insbesondere Brasilin angereichert ist.

Die nachstehenden Untersuchungsergebnisse dienen zur weiteren Erläuterung der Erfindung, ohne diese einzuschränken.

I. Herstellung von Gesamt-Extrakt

Kernholz von Caesalpinia Sappan wurde zerkleinert. In der Rückflußapparatur wurde jeweils 1 Gew.-Teil Holz mit 10 Gew.-Teilen Lösungsmittel ca. 6 h lang extrahiert. Als Lösungsmittel diente Petroläther, Chloroform, Äthanol, Methanol oder Wasser. Anschließend wurde das Lösungsmittel durch Abdampfen entfernt. Bei Bedarf kann die einmal extrahierte Holzmasse ein zweites oder ein drittes Mal extrahiert werden. Der erhaltene trockene,rotbraune Rückstand wurde pulverisiert und gewogen. Es wurden die aus nachstehender Tabelle 1 ersichtlichen Ausbeuten erhalten, wobei sich die Prozentangaben auf die eingesetzte Holzmenge beziehen.

T a b e l l e 1

| Extraktion mit | Ausbeuten an Gesamtextrakt (%) |
|---|---|
| Petroläther | 0,08 |
| Chloroform | 0,11 |
| Äthanol | 2,50 |
| Methanol | 3,00 |
| Wasser | 7,30 |

Die Fig. 1 und 2 zeigen IR-Spektren (des KBr-Preßlings) des Petroläther-Gesamtextraktes (Fig. 1) und des Wasser-Gesamtextraktes (Fig. 2) im Wellenzahlenbereich zwischen 3000 und 800 $cm^{-1}$.

Weitere Holzproben wurden mit siedendem Wasser extrahiert und das Lösungsmittel durch Gefriertrocknung entfernt. In diesem Falle fiel der Rückstand bereits als feines Pulver an, so daß eine gesonderte Pulverisierung unterbleiben konnte.

Die erhaltenen Rückstände wurden mittels verschiedener chromatographischer Verfahren (Dünnschicht-Chromatographie, Säulen-Chromatographie, Hochdruck-Flüssigkeits-Chromatographie (HPLC), Gelfiltration und dergleichen) weiter fraktioniert, wobei stets mit einem weniger polaren Lösungsmittel begonnen und dann stärker polare Lösungsmittel eingesetzt wurden. Durch Vergleich mit handelsüblich zugänglichen Proben von Brasilin und Hämatoxylin konnte die Anwesenheit dieser Substanzen im Gesamtextrakt nachgewiesen werden.

Bei den nachstehenden Untersuchungen wurde der bei der Extraktion von Holz von Caesalpinia Sappan mit Wasser erhaltene Gesamtextrakt eingesetzt. Nach bekannten galenischen Verfahren wurden aus dem pulverförmigen, rotbraunen Rückstand Kapseln mit einem Gesamtextraktgehalt von 250 mg oder 500 mg hergestellt, deren Hülle sich im Magen-Darm-Trakt auflöst.

II. Klinische Beobachtungen

Fall 1:

Der Patient (männlich, Alter: 50 Jahre, Größe: 166 cm, Gewicht: 66 kg) wurde seit einem Jahr wegen Diabetes mellitus behandelt. Er erhielt ein orales, hypoglykämisches Mittel (Diabines, ein Sulfonylharnstoff-Derivat). 14 Tage lang wurden zusätzlich 3 $^{x}$ täglich 500 mg Gesamtextrakt verabreicht. Innerhalb des sich über 44 Wochen erstreckenden Beobachtungszeitraums wurden fortlaufend 3 $^{x}$ täglich 500 mg Gesamtextrakt verabreicht. Der Verlauf der Blutzuckerwerte ergibt sich aus nachstehender Tabelle 2 (wobei FBS für den Nüchtern-Blutzuckerwert und PC2hBS für den post-prandialen Blutzuckerwert 2 h nach der Nahrungsaufnahme entstehen).

## Tabelle 2

| Wochen | 0 | 2 | 6 | 8 | 10 | 12 | 14 | 16 |
|---|---|---|---|---|---|---|---|---|
| FBS | 90 | 120 | | 90 | 105 | | | |
| PC2hBS | 100 | 130 | 95 | | 110 | 115 | 130 | 85 |
| Wochen | 18 | 20 | 22 | 24 | 26 | 28 | 30 | 32 |
| FBS | 120 | 110 | 90 | | | 60 | | |
| PC2hBS | | | | 100 | 96 | | 108 | 124 |
| Wochen | 34 | 36 | 38 | 40 | 42 | 44 | | |
| FBS | 95 | | | 98 | | 80 | | |
| PC2hBS | | 110 | 110 | | 118 | 105 | | |

Im Verlauf des Beobachtungszeitraumes hatte sich auch der Glukosetoleranztest (GTT) deutlich gebessert.

Fall 2:

Der Patient (männlich, Alter: 38 Jahre, Größe 171 cm, Gewicht: 62 kg) litt seit wenigstens einem Jahr an Diabetes mellitus und wurde mit Insulin behandelt. Die Dosis betrug zu Beginn des Beobachtungszeitraums 56 Einheiten NpH-Insulin, wurde laufend verringert (in der 2. Woche 32 Einheiten, in der 4. Woche 16 Einheiten, in der 6. Woche 8 Einheiten) und schließlich in der 8. Woche völlig abgesetzt. Im Verlauf des gesamten, 24 Wochen umfassenden Beobachtungszeitraums erhielt der Patient 3 x täglich 250 mg Gesamtextrakt. Der Verlauf der Blutzuckerwerte ergibt sich aus nachstehender Tabelle 3.

## Tabelle 3

| Wochen | 0 | 2 | 4 | 6 | 14 | 16 | 18 | 12 | 24 |
|---|---|---|---|---|---|---|---|---|---|
| FBS | 105 | | 110 | 90 | 115 | | 115 | 140 | 115 |
| PC2hBS | 220 | 190 | 160 | 130 | | 210 | 125 | | |

Fall 3:

Der Patient (männlich, Alter: 40 Jahre, Größe: 170 cm, Gewicht: 66 kg) litt seit wenigstens fünf Jahren an Diabetes mellitus. Anfänglich wurde 18 Wochen lang als einziges Medikament Gesamtextrakt verabreicht. In der 18. Woche wurde das Präparat abgesetzt. Nachfolgeuntersuchungen erfolgten in der 40. und 54. Woche. Die ermittelten Blutzuckerwerte sind in der nachstehenden Tabelle 4 aufgeführt.

Tabelle 4

| Wochen | 0 | 2 | 4 | 6 | 8 | 10 | 12 | 14 | 16 | 40 | 54 |
|---|---|---|---|---|---|---|---|---|---|---|---|
| FBS | 195 | 160 | 115 | 125 | 90 | 125 | 80 | 100 | 90 | 85 | 110 |
| PC2hBS | 380 | 205 | 110 | 210 | 90 | 120 | 115 | | | | |

Die Fig. 3 zeigt in graphischer Darstellung für diesen Patienten die Auswertung des Glukosetoleranztestes (GTT) in der 1., 15. und 40. Woche.

Fall 4:

Der Patient (männlich, Alter: 46 Jahre, Größe: 173 cm, Gewicht: 58 kg) war seit wenigstens einem Jahr an Diabetes mellitus erkrankt. Anfänglich wurde 24 Wochen lang als einziges Medikament 3 x täglich 500 mg Gesamtextrakt verabreicht. Daraufhin wurde das Präparat abgesetzt, und im Verlauf der anschließenden 1,5 Jahre in regelmäßigen Abständen Nachfolgeuntersuchungen durchgeführt. Die ermittelten Blutzuckerwerte sind in der nachstehenden Tabelle 5 aufgeführt.

Tabelle 5

| Wochen | 0 | 2 | 4 | 6 | 8 | 10 | 12 | 14 | 16 | 18 |
|---|---|---|---|---|---|---|---|---|---|---|
| FBS | 160 | 120 | 110 | 110 | 90 | 90 | 105 | 85 | | 90 |
| PC2hBS | 170 | | | | | | 95 | | 100 | |

| Wochen | 20 | 22 | 26 | 40 | 56 | 72 | 88 | 104 |
|---|---|---|---|---|---|---|---|---|
| FBS | 95 | 80 | 100 | 80 | 90 | 90 | 80 | 80 |
| PC2hBS | | | 100 | | | | | |

Fall 5:

Der Patient (männlich, Alter: 41 Jahre, Größe: 174 cm, Gewicht: 54 kg) war seit wenigstens vier Jahren an Diabetes mellitus erkrankt. 12 Wochen lang wurde als einziges Medikament 3 x täglich 500 mg Gesamtextrakt verabreicht. Danach wurde das Präparat abgesetzt und in regelmäßigen Abständen Nachfolgeuntersuchungen bis zur 35. Woche durchgeführt. Die ermittelten Blutzuckerwerte sind in der nachstehenden Tabelle 6 aufgeführt.

Tabelle 6

| Wochen | 0 | 2 | 4 | 8 | 10 | 16 | 22 | 28 | 35 |
|---|---|---|---|---|---|---|---|---|---|
| FBS | 145 | | 120 | 105 | 90 | 100 | 110 | 80 | 105 |
| PC2hBS | 200 | 115 | 140 | | 100 | 105 | 105 | | |

Bis zur 28. Woche war das Gewicht des Patienten von ursprünglich 54 kg auf 65 kg angestiegen und hielt sich dann weiter auf diesem Wert.

Die Fig. 4 zeigt für diesen Patienten in graphischer Darstellung die Auswertung des Glukosetoleranztestes (GTT) in der 1., 12. und 34. Woche.

Fall 6:

Der Patient (männlich, Alter: 34 Jahre, Größe: 165 cm, Gewicht: 55 kg) war seit wenigstens einem Jahr an Diabetes mellitus erkrankt. Während des 22 Wochen umfassenden Beobachtungszeitraumes wurde als einziges Medikament täglich 3 × 500 mg Gesamtextrakt verabreicht. Die ermittelten Blutzuckerwerte sind in der nachstehenden Tabelle 7 aufgeführt.

Tabelle 7

| Wochen | 0 | 2 | 4 | 6 | 8 | 10 | 12 | 14 | 16 | 18 | 20 | 22 |
|---|---|---|---|---|---|---|---|---|---|---|---|---|
| FBS | 155 | 150 | 150 | 140 | 130 | 120 | 105 | 100 | 130 | 105 | 115 | 110 |
| PC2hBS | 340 | | | | 220 | | | 195 | | | 135 | 110 |

Im Verlauf des Beobachtungszeitraums war das Gewicht des Patienten von anfänglich 55 kg auf 60 kg angestiegen.

Beachtenswert ist die außerordentlich rasche Normalisierung der Blutzuckerwerte.

III. Blutzucker-senkende Wirkung von Brasilin

Durch intraperitoneale Verabreichung von Streptozotocin (70 mg pro 1 kg Körpergewicht in 0,1 M Citratpuffer-Lösung, pH 4,0) wurde an SD-Ratten Diabetes induziert. 20 Tage nach der Streptozotokin-Injektion wurden die Blutzuckerwerte der Versuchstiere gemessen. Die hyperglykämischen Ratten wurden in zwei Gruppen eingeteilt, nämlich in eine Kontrollgruppe und in eine Versuchsgruppe. Den Tieren der Versuchsgruppe wurden zehn Tage lang täglich 110 mg Brasilin pro kg Körpergewicht verabreicht. Anschließend wurden erneut die Blutzuckerwerte gemessen. Die Ergebnisse sind in nachstehender Tabelle 8 aufgeführt.

Tabelle 8

| Blutzucker (mg/100 ml) | | |
|---|---|---|
| | 20 Tage nach Diabetesbeginn | 30 Tage nach Diabetesbeginn |
| diabetische Ratten | 280 ± 26,8 | 337 ± 45,6 |
| diabetische, mit Brasilin behandelte Ratten | 266 ± 16,7 | 165 ± 42,7 |
| normale Ratten | | 79 ± 22,2 |
| normale, mit Brasilin behandelte Ratten | | 77 ± 20,3 |

Ersichtlich führte die Brasilinbehandlung zu einer signifikanten Senkung der Blutzuckerwerte.

IV. Verstärkung der peripheren Insulinwirkung

Diabetisch eingestellte Mäuse wurden in eine Kontroll-und in eine Versuchsgruppe eingeteilt. Den Tieren der Versuchsgruppe wurde täglich 100 mg Brasilin pro kg Körpergewicht intraperitoneal verabreicht. Den Tieren der Kontrollgruppe wurde stattdessen die gleiche Menge Kochsalzlösung intraperitoneal injiziert.

Nach 30 Versuchstagen war das Körpergewicht der Tiere der Versuchsgruppe höher und deren Blutzuckerwerte niedriger im Vergleich zu den diabetisch eingestellten Tieren der Kontrollgruppe.

Am 30. Versuchstag ließ man die Tiere 5 h lang fasten und injizierte daraufhin intravenös 0,46 mU/g NPH-Insulin. Daraufhin wurden im Abstand von je 5 min die Blutzuckerwerte bestimmt.

Die ermittelten Ergebnisse sind graphisch in Fig. 5 dargestellt (jeder dargestellte Punkt entspricht einem Mittelwert aus fünf Messungen).

Ersichtlich vermag diese geringe NPH-Insulin-Gabe den Blutzuckerwert der diabetischen Mäuse - (Kontrollgruppe) nicht signifikant zu verändern. Demgegenüber wurde an den Mäusen der Versuchsgruppe (mit Brasilin behandelt) eine deutliche Abnahme der Blutzuckerwerte festgestellt.

Zur Deutung dieses Befundes wurden weitere Untersuchungen durchgeführt mit dem Ziel, festzustellen, ob Brasilin Affinität der Insulinrezeptoren verändert oder gegebenenfalls die Anzahl der Insulinrezeptoren zu steigern vermag.

Den Versuchstieren der oben bezeichneten Kontrollgruppe (diabetische Mäuse) und der Versuchsgruppe (mit Brasilin behandelte diabetische Mäuse) wurden Eryhtrocyten ($2,6 \times 10^9$/ml) entnommen, an diese mit Jod-125 markiertes Insulin gebunden (85 pM$^{125}$J) und daraufhin in Gegenwart von steigenden Mengen nicht markiertem Insulin - (32 pM bis 1,8 $\mu$M) 120 min lang bei 15°C inkubiert. Durch Subtraktion des nicht-spezifisch gebundenen Insulins (10 ng/ml) wurde daraufhin der Anteil an spezifisch gebundenem $^{125}$Jod-Insulin bestimmt. Die erhaltenen Ergebnisse sind graphisch in Fig. 6 dargestellt. Die Fig. 7 zeigt die aus den Bindungswerten gebildete Scachard-Analyse.

Aus dem weitgehend ähnlichen Verlauf der Abnahme des spezifisch gebundenen, markierten Insulins für die Versuchstiere der Kontrollgruppe und der Versuchsgruppe muß geschlossen werden, daß keine, zumindest keine wesentlichen Affinitätsunterschiede vorliegen, sondern daß die bei Brasilinbehandlung auftretende, gesteigerte periphere Insulinwirkung auf einer Erhöhung der Anzahl der peripheren Insulinrezeptoren beruht.

Hierin wird die Ursache gesehen, daß bei zusätzlicher Verabreichung von Brasilin oder Gesamtextrakt die von einem Diabetiker benötigte Insulingabe verringert werden kann. In diesem Sinne kann Brasilin oder Gesamtextrakt als Ersatz für die bekannten Sulfonylharnstoff-Derivate oder als Ergänzung bei der Behandlung mit hypoglykämischen Mitteln, etwa auf der Basis von Sulfonylharnstoff-Derivaten dienen.

V. Wirkung von Brasilin auf Postrezeptor-Störungen

Bei Insulin-abhängigem Diabetes mellitus tritt häufig eine Insulinresistent auf. Auch bei, durch Verabreichung von Streptozotocin diabetisch eingestellten Ratten wird häufig eine verminderte Insulinwirksamkeit beobachtet. Für eine solche Insulinresistenz werden insbesondere auch Postrezeptor-Störungen verantwortlich gemacht.

Untersucht wurde an (a) unbehandelten - (normalen) Kontrolltieren (Ratten), (b) diabetisch eingestellten Ratten und (c) diabetisch eingestellten und mit Brasilin behandelten Ratten die Glukoseoxidation und die Fettbildung (anhand der veresterten Fette und der freien Fettsäuren). Diabetes wurde durch intravenöse Verabreichung von Streptozotocin (40 mg/kg Lebendgewicht) induziert. Brasilin wurde 15 Tage lang (100 mg/kg Körpergewicht) intraperitoneal verabreicht. Die unbehandelten Tiere der Kontrollgruppe erhielten in der gleichen Zeit ein gleiches Volumen Kochsalzlösung.

Die Auswirkungen der Brasilingabe auf die Glukoseoxidation wurde nach dem von Winegard et.al beschriebenen Verfahren (J. Biol.Chem. 233, 267 - (1958)) unter Verwendung von $^{14}$C markierter Glukose (1-$^{14}$C-Glukose) bestimmt. Die erhaltenen Ergebnisse sind in nachstehender Tabelle 9 aufgeführt.

## Tabelle 9

Bildung von $^{14}CO_2$ in Fettablagerungen aus mit 1-$^{14}$C-Glukose inkubierten Ratten (37°C; 120 min)

| Versuchstiere | Glukose → $CO_2$ (nMol/100mg Gewebe/ 120 min) | |
|---|---|---|
| | Grundzustand | Insulin-Stimulierung* |
| normal | 172,8 ± 21,4 ( 9)** | 694,2 ± 86,0 (9) |
| diabetisch | 109,1 ± 8,4 (10) | 236,6 ± 28,4 (8) |
| diabetisch + Brasilin | 156,6 ± 14,7[a] (8) | 303,1 ± 19,0[b] (8) |

Anmerkungen:

*) Insulinkonzentration: 25 ng/ml
**) Anzahl der Versuchstiere
a) P < 0,01 geg. diabetische Kontrollgruppe
b) P < 0,1 geg. diabetische Kontrollgruppe

Wie aus Tabelle 9 ersichtlich, ist im Grundzustand die Bildung von $CO_2$ in den Fettablagerungen der diabetisch eingestellten Ratten gehemmt. Die zusätzliche Verabreichung von Brasilin vermag bei diesen diabetisch eingestellten Versuchstieren 74,6% der ursprünglichen $CO_2$-Bildung (= Kontrollgruppe) wiederherzustellen. Bei Insulin-Stimulierung ist die Brasilinwirkung geringer. Es werden 14,5% der ursprünglichen $CO_2$-Bildung wiederhergestellt.

Die Auswirkungen einer zusätzlichen Brasilingabe auf die Fettbildung wurde anhand der aus 1-$^{14}$C-Glukose gebildeten veresterten Fetten und freien Fettsäuren bestimmt. Die Messungen erfolgten nach anerkannten Verfahren. Die erhaltenen Ergebnisse sind in den nach folgenden Tabellen 10 und 11 aufgeführt.

## Tabelle 10

Bildung von $^{14}$C-haltigen, veresterten Fetten (E.L.) in Fettablagerungen aus mit 1-$^{14}$C-Glukose inkubierten Ratten (37°C, 120 min)

| Versuchstiere | Glukose → E.L. (nMol/100 mg Gewebe/ 120 min) | |
|---|---|---|
| | Grundzustand | Insulin-Stimulierung* |
| normal | 220,8 ± 11,9 (10)** | 528,0 ± 17,8 (10) |
| diabetisch | 78,8 ± 7,7 (10) | 276,5 ± 20,8 ( 9) |
| diabetisch + Brasilin | 113,9 ± 9,5[a] (10) | 326,2 ± 18,4[b] ( 9) |

Anmerkungen:

*) Insulinkonzentration: 25 ng/ml
**) Anzahl der Versuchstiere
a) P < 0,01 geg. diabetische Kontrollgruppe
b) P < 0,1 geg. diabetische Kontrollgruppe

Ersichtlich wird bei den diabetisch eingestellten Versuchstieren erheblich weniger $^{14}C$ aus der markierten Glukose in den veresterten Fetten gefunden als bei den unbehandelten Versuchstieren der Kontrollgruppe. Die zusätzliche Verabreichung von Brasilin vermag bei den diabetisch eingestellten Versuchstieren immerhin 24,7% der ursprünglichen Bildung von veresterten Fetten aus der markierten Glukose (= Kontrollgruppe) wiederherzustellen. Auch bei Insulin-Stimulierung hat Brasilin einer vergleichbare Wirkung. Es wurden noch 19,8% der ursprünglichen Bildung veresterter Fette wiederhergestellt.

## Tabelle 11

Bildung von $^{14}C$-haltigen freien Fettsäuren (FFA) in Fettablagerungen aus mit 1-$^{14}C$-Glukose inkubierten Ratten (37°C, 120 min)

| Versuchstiere | Glukose → FFA (nMol/100 mg Gewebe/120 min) | |
|---|---|---|
| | Grundzustand | Insulin-Stimulierung* |
| normal | 3,18 ± 0,53 (6)** | 8,42 ± 1,06 (6) |
| diabetisch | 2,60 ± 0,85 (6) | 3,73 ± 0,39 (6) |
| diabetisch + Brasilin | 1,83 ± 0,43[a) ](6) | 3,39 ± 0,76[b)] (6) |

Anmerkungen:

*) Insulinkonzentration: 25 ng/ml
**) Anzahl der Versuchstiere
a), b) keine statistisch signifikanten Unterschiede geg. diabetische Kontrollgruppe

Sowohl im Grundzustand wie bei Insulinstimulierung wird bei den diabetisch eingestellten Versuchstieren weniger $^{14}C$ aus der markierten Glukose in den freien Fettsäuren gefunden als bei den unbehandelten Tieren der Kontrollgruppe. Eine zusätzliche Verabreichung von Brasilin hat in beiden Fällen keine signifikante Auswirkung auf die Bildung freier Fettsäuren.

VI. Beeinflussung des Prostaglandin-Stoffwechsels

1. Plättchenaggregation-hemmende Wirkung von Brasilin und Hämatoxylin

Die Plättchenaggregation wurde mittels eines tubimetrischen Verfahrens in einem Doppelkanal-Aggregometer bestimmt, unter der Annahme, daß ein plättchenarmes Plasma 100%ige Aggregation bedeutet und ein plättchenreiches Plasma 0%ige Aggregation. In jedes Aggregometer-Rohr wurden 40 µl plättchenreiches Plasma und 50 µl Prüflösung eingefüllt. Das Gemisch wurde 2 min lang bei 37°C inkubiert und daraufhin 50 µl ADP-Lösung zugesetzt. Die durch die Prüflösung bedingte prozentuale Hemmung der Plättchenaggregation wurde wie folgt berechnet:

$$\left( 1 - \frac{\text{Inhibitor-induzierte, prozentuale Aggregation}}{\text{ADP-induzierte, prozentuale Aggregation}} \right) \times 100(\%)$$

In der nachstehenden Tabelle 12 sind die Auswirkungen eines Zusatzes von Brasilin oder Hämatoxylin auf die durch 2 µM ADP induzierte Plättchenaggregation an menschlichem diabetischem Plasma aufgeführt.

Tabelle 12

Wirkung von Brasilin und Hämatoxylin auf die durch 2 µM ADP induzierte Plättchenaggregation in diabetischem, menschlichem Plasma

| | Tmax | | | T/min | | |
|---|---|---|---|---|---|---|
| Conc. (M) | Aggregation (%) | | Hemmung (%) | Aggregation (%) | | Hemmung (%) |
| Kontrolle | 78,7 | 100 | | 78,7 | 100 | |
| Brasilin | | | | | | |
| $10^{-3}$ | 46,3 | 58,8 | 41,2 | 8,75 | 11,1 | 88,9 |
| $10^{-4}$ | 70,0 | 88,9 | 11,1 | | | |
| Hämatoxylin | | | | | | |
| $10^{-3}$ | 52,5 | 66,5 | 33,5 | 7,5 | 9,5 | 90,5 |
| $10^{-4}$ | 76,3 | 96,8 | 3,2 | | | |
| Acetylsalicylsäure | | | | | | |
| $10^{-3}$ | 70,0 | 88,9 | 11,1 | | | |
| $10^{-4}$ | 73,8 | 93,6 | 6,4 | | | |

Die nachstehende Tabelle 13 zeigt anhand einer analogen Untersuchung die Auswirkungen von Brasilin und Hämatoxylin auf die Plättchenaggregation in normalem, menschlichem Plasma.

## Tabelle 13

Wirkung von Brasilin und Hämatoxylin auf die durch 2 µM ADP induzierte Plättchenaggregation in normalem, menschlichem Plasma

| | Tmax | | | T/min | | |
|---|---|---|---|---|---|---|
| Conc. (M) | Aggregation (%) | | Hemmung (%) | Aggregation (%) | | Hemmung (%) |
| Kontrolle | 75,1 | 100 | | 75,1 | 100 | |
| Brasilin | | | | | | |
| $10^{-3}$ | 31,5 | 41,8 | 58,2 | 21,1 | 28,1 | 71,9 |
| $10^{-4}$ | 60,7 | 80,1 | 19,9 | 34,8 | 46,4 | 53,6 |
| Hämatoxylin | | | | | | |
| $10^{-3}$ | 22,6 | 30,8 | 69,8 | 11,7 | 15,6 | 84,4 |
| $10^{-4}$ | 68,1 | 90,6 | 9,4 | 58,5 | 78,4 | 21,7 |
| Acetylsalicylsäure | | | | | | |
| $10^{-3}$ | 37,5 | 49,9 | 50,1 | 11,9 | 15,8 | 84,6 |
| $10^{-4}$ | 60,2 | 80,1 | 19,9 | 17,4 | 23,2 | 76,8 |

Die Versuchsergebnisse bestätigen eine erhebliche Hemmung der Plättchenaggregation durch die Anwesenheit von Brasilin oder Hämatoxylin. Ersichtlich wirken Brasilin und Hämatoxylin auf den Prostaglandin-Stoffwechsel ein, vermutlich durch eine hemmende Wirkung auf Zyklooxygenase, Phospholipase $A_2$ und/oder Thromboxan-Synthetase.

2. Hemmung der Malondialdehyd-(MDA)-Bildung durch Brasilin -ohne feststellbaren Einfluß der Prostazyklin-Bildung

Entscheidenden Einfluß auf die Plättchenaggregation hat Thromboxan $A_2$. Das Thromboxan $A_2$ erzeugende Enzymsystem bildet auch MDA. Eine Hemmung der MDA-Bildung kann folgende Ursachen haben:

a) hemmende Wirkung auf Phospholipase $A_2$,

b) hemmende Wirkung auf Zyklooxygenase,

c) hemmende Wirkung auf Thromboxan-Synthetase.

Sofern eine hemmende Wirkung auf MDA nachweisbar ist, und weiterhin kein signifikanter Einfluß auf die Prostazyklin-Bildung nachweisbar ist, kann daraus auf eine selektive, hemmende Wirkung auf Thromboxan $A_2$ geschlossen werden.

Ratten wurden in vier Versuchsgruppen eingeteilt, nämlich

Gruppe A: Übliche Ratten (10 Versuchstiere);

Gruppe B: Übliche Ratten (8 Versuchstiere), denen vom 20. bis 30. Tag des Versuchs täglich wässerige Brasilin-Lösung (mit 110 mg Brasilin pro Körpergewicht) verabreicht wurden;

Gruppe C: Diabetische Ratten (8 Versuchstiere); zur Induzierung der Diabetes wurden üblichen Ratten am ersten Versuchstag pro kg Körpergewicht 70 mg Streptozotocin in 0,1 M Citrat-Puffer-Lösung (pH 4,0) intraperitoneal verabreicht;

Gruppe D: Diabetische Ratten (10 Versuchstiere); zur Induzierung des Diabetes wurden üblichen Ratten am ersten Versuchstag pro kg Körpergewicht 70 mg Streptozotocin in 0,1 M

Citrat-Puffer-Lösung (pH 4,0) intraperitoneal verabreicht. Diesen diabetischen Ratten wurde vom 20. bis 30. Tag des Versuchs täglich wässerige Brasilin-Lösung (mit 110 mg Brasilin pro kg Körpergewicht) oral verabreicht.

(Die Versuchstiere der Gruppen A und C erhielten vom 20. bis 30. Tag anstelle einer wässerigen Brasilin-Lösung die gleiche Menge destilliertes Wasser).

Nach Ablauf des 30. Versuchstages wurden die Versuchstiere geopfert und die MDA-Bildung anhand Thrombinstimulierter Plättchen bestimmt. Die erhaltenen Ergebnisse sind in den nachstehenden Tabellen 14 und 15 aufgeführt.

T a b e l l e 14

| Gruppe | Körpergewicht | Blutzucker (mg/100 ml) | | MDA (nMol/10 Plättchen) | | |
|---|---|---|---|---|---|---|
| | am 30.Tag | am 20.Tag | am 30.Tag | Thrombin 5,0I.Ein. | Thrombin 1,0I.Ein. | Thrombin 0,2I.Ein. |
| A | 222±17,8 (g) | | 79±22,2 | 5,16±2,44 | 2,46±0,92 | 4,16±2,04 |
| B | 226±24,4 (g) | | 77±20,3 | 2,20±0,58 | 1,02±0,48 | 0,58±0,30 |
| C | 185±24,4 (g) | 280±26,8 | 337±45,6 | 6,26±1,36 | 3,38±0,71 | 2,86±0,36 |
| D | 206±9,61 (g) | 266±16,7 | 165±42,7 | 2,22±0,35 | 1,10±0,31 | 0,76±0,21 |

T a b e l l e 15

| Versuchstiere (Ratten) | G-Keto PG F. (ng/mg) |
|---|---|
| normal | 0,18 ± 0,03 |
| normal + Brasilin | 0,17 ± 0,02 |
| diabetisch | 0,19 ± 0,02 |
| diabetisch + Brasilin | 0,18 ± 0,03 |

Ersichtlich hat Brasilin eine hemmende Wirkung auf die MDA-Bildung, wobei kein Einfluß auf die Prostazyklin-Bildung festgestellt werden konnte.

VII. Normalisierende Wirkung auf lysosomale Enzyme

Die in der Niere aktiven lysosomalen Glykosidasen sind möglicherweise bei der mit Diabetes mellitus einhergehenden Mikroangiopathie involviert. Es wurden deshalb die Aktivitäten der lysosomalen Glykosidasen bei der Verabreichung von Brasilin und Gesamtextrakt untersucht.

Eine Anzahl Mäuse wurde in drei Gruppen eingeteilt. Die als Kontrollgruppe dienende Gruppe I blieb unbehandelt. An den restlichen Mäusen wurde durch Verabreichung von Alloxan Diabetes induziert. Ein Teil dieser diabetischen Mäuse blieb unbehandelt (diabetische Kontrollgruppe II); dem restlichen Teil der diabetischen Mäuse wurde täglich 100 mg Brasilin pro kg Körpergewicht verabreicht. Nach einer Versuchsdauer von zehn Tagen bzw. 30 Tagen wurde den Versuchstieren Nierengewebe entnommen und die Enzymfraktion bestimmt. Im einzelnen wurde 30 min lang bei 37°C inkubiert, die Reaktion durch Zusatz von 0,05 M Glycin-NaOH-Pufferlösung (pH 10,4) gestoppt, 10 min lang bei 3000 U/min zentrifugiert und am Überstand spektrophotometrisch bei 410 nm die gebildete Menge p-Nitrophenol pro min und pro mg Protein bestimmt. Hierbei wurden die aus nachstehender Tabelle 16 ersichtlichen Ergebnisse erhalten.

## Tabelle 16

| Dauer der Behandlung | Enzym | Gruppe I | Gruppe II | Gruppe III |
|---|---|---|---|---|
| nach 10 Tagen | N-acetyl-ß-D-glucosaminidase | 64,51±4,59 | 52,93±3,55[b] | 60,68±3,84[D] |
| | -Mannosidase | 8,72±1,01 | 8,60±1,51 | 12,49±1,34[A] |
| | ß -Galactosidase | 11,53±2,81 | 14,74±1,97[e] | 12,33±1,95[E] |
| | -Glucosidase | 4,05±0,59 | 3,63±0,71 | 4,62±0,75[D] |
| | ß -Glucosidase | 1,25±0,13 | 1,49±0,31[f] | 1,86±0,30[E] |
| | ß -Glucuronidase | 4,82±0,69 | 3,33±0,41[a] | 4,02±0,65[D] |
| nach 30 Tagen | N-acetyl-ß-D-glucosaminidase | 65,11±5,37 | 57,95±2,25[c] | 67,79±4,06[A] |
| | -Mannosidase | 6,57±0,15 | 5,69±0,32[a] | 5,99±0,54[A] |
| | ß-Galactosidase | 8,90±1,26 | 7,39±1,35[e] | 10,28±0,55[A] |
| | -Glucosidase | 3,76±0,62 | 3,33±0,36 | 4,12±0,40[D] |
| | ß-Glucosidase | 2,54±0,35 | 2,74±0,21 | 2,95±0,20[E] |
| | ß-Glucoronidase | 2,26±0,21 | 2,31±0,13 | 2,49±0,25[E] |

Anmerkungen:

a-f; Gruppe I gegenüber Gruppe II

A-F; Gruppe II gegenüber Gruppe III

a,A; P < 0,001
b,B; P < 0,005
c,C; P < 0,01
d,D; P < 0,025
e,E; P < 0,05
f,F; P < 0,1

Ersichtlich hat die Verabreichung von Brasilin eine normalisierende Wirkung auf die Aktivität der lysosomalen Glykosidasen. Dieser Aspekt der biologischen Aktivität von Brasilin kann eine wichtige Rolle bei der Verhinderung der diabetischen Mikroangiopathie spielen.

Ähnliche Ergebnisse werden auch bei der Verabreichung von Gesamtextrakten aus verschiedenen Pflanzen der Gattung Caesalpinia erhalten. Ferner wurden diese Wirkungen nicht nur bei Alloxan induzierten, diabetischen Mäusen, sondern auch bei Streptozotokin induzierten sowie bei spontan diabetischen Mäusen festgestellt.

VIII. Senkung der Blutviskosität und Erhöhung der Erythrozyten-Verformbarkeit

Eine erhöhte Blutviskosität und eine verringerte Erythrozyten-Verformbarkeit sind typische pathologische Zustände diabetischer Subjekte. Untersucht wurde die Auswirkung einer Verabreichung von Brasilin an diabetisch eingestellten Versuchstieren (Ratten) auf deren Blutviskosität und deren Erythrozyten-Verformbarkeit.

Die Versuchstiere wurden in vier Gruppen eingeteilt, nämlich

(a) unbehandelte Versuchstiere (normal);

(b) lediglich mit Brasilin behandelte Versuchstiere (normal + Brasilin);

(c) lediglich diabetisch eingestellte Versuchstiere (diabetisch); und

(d) diabetisch eingestellte und mit Brasilin behandelte Versuchstiere (diabetisch + Brasilin).

Zur Verabreichung von Brasilin wurde den Versuchstieren zehn Tage lang pro Tag jeweils 100 mg Brasilin pro kg Körpergewicht intraperitoneal injiziert. Zur Einstellung des Diabetes wurden 175 mg Alloxan pro kg Körpergewicht intraperitoneal verabreicht.

Die Bestimmung der Blutviskosität erfolgte an heparinisierten Blutproben relativ zu destilliertem Wasser bei 37 ± 0,5°C. Es wurde die Zeitspanne bestimmt, die zum Durchlaufen von 2 ml Flüssigkeit zwischen zwei Markierungen an einer engen Glaskapillare erforderlich war. Die dabei erhaltenen Ergebnisse sind in der nachstehenden Tabelle 17 aufgeführt.

Tabelle 17

Auswirkung von Brasilin auf die Blutviskosität von unbehandelten und diabetisch eingestellten Ratten.

| Versuchstiere | Viskosität (cP) |
|---|---|
| normal | 1,45[a] ± 0,08 |
| normal + Brasilin | 1,40 ± 0,06 |
| diabetisch | 1,62 ± 0,09[b] |
| diabetisch + Brasilin | 1,52 ± 0,07[c] |

Anmerkungen:

a) Jeder angegebene Wert ist ein aus sechs Meßwerten ermittelter Mittelwert;

b) P < 0,01 geg. normale Kontrollgruppe;

c) P < 0,05 geg. diabetische Kontrollgruppe.

Ersichtlich ist bei den diabetisch eingestellten Versuchstieren die Blutviskosität signifikant erhöht (P < 0,01). Im Vergleich zwischen unbehandelten Versuchstieren (normal) und lediglich mit Brasilin behandelten Tieren wurden keine statistischen Unterschiede hinsichtlich der Blutviskosität festgestellt. Jedoch ergab sich die Brasilinbehandlung der diabetisch eingestellten Versuchstiere eine signifikante Abnahme (P < 0,05) der Blutviskosität. Diese Ergebnisse berechtigen zu dem Schluß, daß eine Langzeitbehandlung von Diabetikern mit Brasilin eine normalisierende, d.h. senkende Wirkung auf deren typischerweise pathologisch erhöhte Blutviskosität hat.

Zur Bestimmung der Erythrozyten-Verformbarkeit wurde unter üblichen, standardisierten Bedingungen die Filtriergeschwindigkeit von Blut der verschiedenen Versuchstiere bestimmt, das etwa 4,9 $\times 10^{12}$ Erythrozyten pro Liter enthielt. Die dabei erhaltenen Ergebnisse sind in der nachstehenden Tabelle 18 aufgeführt.

T a b e l l e 18

Auswirkung von Brasilin auf die Erythrozyten-Verformbarkeit von unbehandelten und diabetisch eingestellten Ratten

| Versuchstiere | Filtriergeschwindigkeit (ml/min) |
|---|---|
| normal | 22,9[a] ± 0,3 |
| normal + Brasilin | 23,1 ± 0,3 |
| diabetisch | 17,4 ± 0,1[b] |
| diabetisch + Brasilin | 19,6 ± 0,4[c] |

Anmerkungen:

a) Jeder angegebene Wert ist ein aus sechs Meßwerten ermittelter Mittelwert;

b) P < 0,01 geg. unbehandelte Kontrollgruppe;

c) P < 0,01 geg. unbehandelte und geg. diabetische Kontrollgruppe.

Es wurde sichergestellt, daß Brasilin keinen Einfluß auf die Erythrozyten-Bildung hat. Das Blut der mit Alloxan diabetisch eingestellten Ratten zeigte eine signifikant geringere Filtriergeschwindigkeit (P < 0,01) als dasjenige der unbehandelten Ratten, was -im Hinblick auf die Versuchsbedingungen -als eine verminderte Erythrozyten-Verformbarkeit gewertet werden muß. Die Brasilinbehandlung hatte keinen signifikanten Einfluß auf die Filtriergeschwindigkeit des Blutes der ansonsten unbehandelten Tiere. Jedoch bewirkte die Brasilinbehandlung bei den diabetisch eingestellten Tieren eine signifikante Steigerung der Filtriergeschwindigkeit, was als Hinweis auf eine höhere Erythrozyten-Verformbarkeit gewertet werden muß. Diese Ergebnisse berechtigen zu dem Schluß, daß eine Langzeitbehandlung von Diabetikern mit Brasilin eine normalisierende, d.h. erhöhende Wirkung auf deren typischerweise pathologisch verminderte Erythrozyten-Verformbarkeit hat.

**Ansprüche**

1. Arzneimittel,

dadurch gekennzeichnet, daß

das Arzneimittel als Wirkstoff einen aus Pflanzenmaterial der zur Gattung Caesalpinia gehörenden Pflanzen extrahierbaren Extrakt, eine oder mehrere Komponente(n) dieses Extraktes und/oder eine oder mehrere in diesem Extrakt vorkommende oder davon abgeleitete, jedoch synthetisch hergestellte Substanz(en) enthält.

2. Ein Arzneimittel, mit einer

-vorbeugenden und/oder heilenden Wirkung auf mikrozirkulatorische Störungen,

-antidiabetischen Wirkung,

-Blutzucker-senkenden Wirkung,

-die Plättchenaggregation hemmenden Wirkung,

-die Blutviskosität senkenden Wirkung,

-die Erythrozyten-Verformbarkeit steigernden Wirkung,

-normalisierenden Wirkung auf die lysosomalen Enzyme, und/oder mit einer

-den Prostaglandin-Stoffwechsel beeinflußenden, hier insbesondere selektiv

Thromboxan $A_2$ hemmenden Wirkung,

dadurch gekennzeichnet, daß

das Arzneimittel als Wirkstoff einen aus Pflanzenmaterial der zur Gattung Caesalpinia gehörenden Pflanzen extrahierbaren Extrakt, eine oder mehrere Komponente(n) dieses Extraktes und/oder eine oder mehrere in diesem Extrakt vorkommende oder davon abgeleitete , jedoch synthetisch hergestellte Substanz(en) enthält.

3. Ein gegen Diabetes mellitus wirksames Arzneimittel,

dadurch gekennzeichnet, daß

das Arzneimittel als Wirkstoff einen aus Pflanzenmaterial der zur Gattung Caesalpinia gehörenden Pflanzen extrahierbaren Extrakt, eine oder mehrere Kompo nente(n) dieses Extraktes und/oder eine oder mehrere in diesem Extrakt vorkommende oder davon abgeleitete, jedoch synthetisch hergestellte Substanz(en) enthält.

4. Das Arzneimittel nach einem der Ansprüche 1 bis 3,

dadurch gekennzeichnet, daß

der Extrakt aus Holz von Caesalpinia Sappan, von Hämatoxylon Campechianum, von Caesalpinia Echinata, von Caesalpinia Coriaria, von Caesalpinia Brasiliensis und/oder von Hämatoxylon Braziletto erhältlich ist.

5. Das Arzneimittel nach einem der Ansprüche 1 bis 4,

dadurch gekennzeichnet, daß

der Extrakt aus Holz von Caesalpinia Sappan erhältlich ist.

6. Das Arzneimittel nach einem der Ansprüche 1 bis 5,

dadurch gekennzeichnet, daß

der Extrakt durch mehrstündiges Auskochen des Holzes mit Wasser erhältlich ist.

7. Das Arzneimittel nach einem der Ansprüche 1 bis 6,

dadurch gekennzeichnet, daß

der Wirkstoff ein Benz(b)indeno(2.1-d)pyran-Derivat entsprechend nachstehender allgemeiner Strukturformel (I) ist

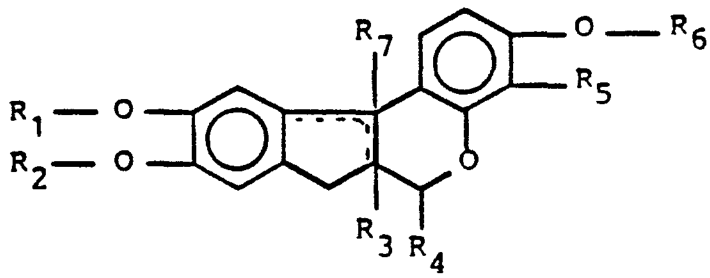

(I)

wobei

$R_1$, $R_2$ und $R_6$ gleich oder verschieden sein können und stehen für einen Wasserstoffrest, einen niederen Alkylrest, vorzugsweise Methylrest, einen Acetylrest, einen Benzylrest, oder $R_1$ und $R_2$ gemeinsam eine Methylengruppe bilden;

$R_3$ und $R_7$ gleich oder verschieden sein können und stehen für einen Wasserstoffrest, Hydroxylrest oder Acetylrest;

$R_4$ für einen Wasserstoffrest oder eine Oxogruppe steht;

$R_5$ für einen Wasserstoffrest, einen Hydroxylrest, einen niederen Alkylrest, vorzugsweise Methylrest, einen Acetylrest oder einen Benzylrest steht; und

- - - - - - - - für eine Einfachbindung oder für eine Doppelbindung steht.

8. Das Arzneimittel nach einem der Ansprüche 1 bis 6,

dadurch gekennzeichnet, daß

der Wirkstoff ein Benz(b)indeno(2.1-d)pyran-Derivat entsprechend nachstehender allgemeiner Strukturformel (II) ist

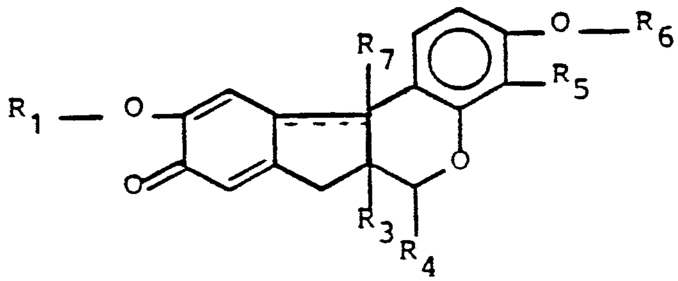

(II)

wobei

$R_1$ und $R_6$ gleich oder verschieden sein können und stehen für einen Wasserstoffrest, einen niederen Alkylrest, vorzugsweise Methylrest, einen Acetylrest, einen Benzylrest, oder

$R_1$ und $R_6$ gemeinsam eine Methylengruppe bilden;

$R_3$ und $R_7$ gleich oder verschieden sein können und stehen für einen Wasserstoffrest, Hydroxylrest oder Acetylrest;

$R_4$ für einen Wasserstoffrest oder eine Oxogruppe steht;

$R_5$ für einen Wasserstoffrest, einen Hydroxylrest, einen niederen Alkylrest, vorzugsweise Methylrest, einen Acetylrest oder einen Benzylrest steht; und

für eine Einfachbindung oder für eine Doppelbindung steht.

9. Das Arzneimittel nach einem der Ansprüche 1 bis 6,

dadurch gekennzeichnet, daß

der Wirkstoff Brasilin oder Brasilein ist.

10. Das Arzneimittel nach einem der Ansprüche 1 bis 6,

dadurch gekennzeichnet, daß

der Wirkstoff Hämatoxylin und/oder Hämatein ist.

11. Ein Verfahren zur Herstellung eines Arzneimittels mit einer

-vorbeugenden und/oder heilenden Wirkung auf mikrozirkulatorische Störungen,

-antidiabetischen Wirkung,

-Blutzucker-senkenden Wirkung,

-die Plättchenaggregation hemmenden Wirkung,

-die Blutviskosität senkenden Wirkung,

-die Erythrozyten-Verformbarkeit steigernden Wirkung,

-normalisierenden Wirkung auf die lysosomalen Enzyme und/oder mit einer

-den Prostaglandin-Stoffwechsel beeinflußenden, hier insbesondere selektiv

Thromboxan $A_2$ hemmenden Wirkung,

dadurch gekennzeichnet, daß

Pflanzenmaterial der zur Gattung Caesalpinia gehörenden Pflanzen mit einem Lösungsmittel extrahiert, das Lösungsmittel entfernt, und

der verbleibende feste Rückstand zu einem Arzneimittel konfektioniert wird.

12. Das Verfahren nach Anspruch 11,

dadurch gekennzeichnet , daß

Holz von Bäumen der Gattung Caesalpinia extrahiert wird, zu deren Inhaltsstoffen Brasilin und/oder Hämatoxylin gehören.

13. Das Verfahren nach Anspruch 11 oder 12,

dadurch gekennzeichnet, daß

Holz von Caesalpinia Sappan, von Hämatoxylon Campechianum, von Caesalpinia Echinata, von Caesalpinia Coriaria, von Caesalpinia Brasiliensis und/oder von Hämatoxylon Braziletto extrahiert wird.

14. Das Verfahren nach einem der Ansprüche 11 bis 13,

dadurch gekennzeichnet, daß

Holz von Caesalpinia Sappan extrahiert wird.

15. Das Verfahren nach einem der Ansprüche 11 bis 14,

dadurch gekennzeichnet, daß

das Pflanzenmaterial, insbesondere Holz, mit einem Lösungsmittel, ausgewählt aus nachstehender Gruppe, extrahiert wird, nämlich Wasser, niedere Alkohole, Diäthyläther, höhersiedende Äther, aliphatische Ketone, aliphatische Kohlenwasserstoffe, chlorierte Kohlenwasserstoffe oder Äthylacetat.

16. Das Verfahren nach einem der Ansprüche 11 bis 15,

dadurch gekennzeichnet, daß

das Pflanzenmaterial, insbesondere Holz, mit Wasser extrahiert wird.

17. Das Verfahren nach einem der Ansprüche 11 bis 16,

dadurch gekennzeichnet, daß

das Pflanzenmaterial, insbesondere Holz, mehrere Stunden lang mit einem siedenden Lösungsmittel extrahiert wird.

18. Das Verfahren nach einem der Ansprüche 11 bis 17,

dadurch gekennzeichnet, daß

mehrere Extrakte, die durch Extrahieren von Pflanzenmaterial von verschiedenen, zur Gattung Caesalpinia gehörenden Pflanzen isoliert worden sind, miteinander vermischt werden.

19. Das Verfahren nach einem der Ansprüche 11 bis 18,

dadurch gekennzeichnet, daß

der Extrakt mit einem Benz(b)indeno(2.1.-d)pyran-Derivat entsprechend nachstehender allgemeiner Strukturformel (I) angereichert wird,

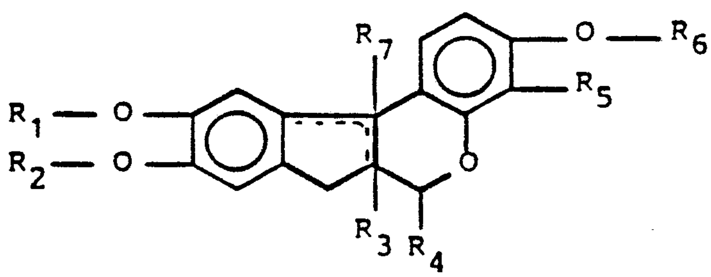

(I)

wobei

$R_1$, $R_2$ und $R_6$ gleich oder verschieden sein können und stehen für einen Wasserstoffrest, einen niederen Alkylrest, vorzugsweise Methylrest, einen Acetylrest, einen Benzylrest, oder $R_1$ und $R_2$ gemeinsam eine Methylengruppe bilden;

$R_3$ und $R_7$ gleich oder verschieden sein können und stehen für einen Wasserstoffrest, Hydroxylrest oder Acetylrest;

$R_4$ für einen Wasserstoffrest oder eine Oxogruppe steht;

$R_5$ für einen Wasserstoffrest, einen Hydroxylrest, einen niederen Alkylrest, vorzugsweise Methylrest, einen Acetylrest oder einen Benzylrest steht, und

für eine Einfachbindung oder für eine Doppelbindung steht.

20. Das Verfahren nach einem der Ansprüche 11 bis 18,

dadurch gekennzeichnet, daß

der Extrakt mit einem Benz(b)indeno(2.1-d)pyran-Derivat entsprechend nachstehender allgemeiner Strukturformel (II) angereichert wird,

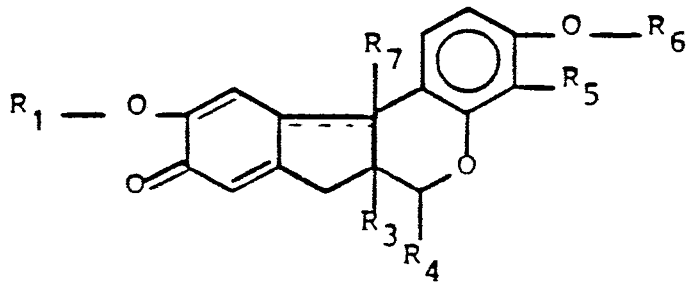

(II)

wobei

$R_1$ und $R_6$ gleich oder verschieden sein können und stehen für einen Wasserstoffrest, einen niederen Alkylrest, vorzugsweise Methylrest, einen Acetylrest, einen Benzylrest, oder

$R_1$ und $R_6$ gemeinsam eine Methylengruppe bilden;

$R_3$ und $R_7$ gleich oder verschieden sein können und stehen für einen Wasserstoffrest, Hydroxylrest oder Acetylrest;

$R_4$ für einen Wasserstoffrest oder eine Oxogruppe steht;

$R_5$ für einen Wasserstoffrest, einen Hydroxylrest, einen niederen Alkylrest, vorzugsweise Methylrest, einen Acetylrest oder einen Benzylrest steht; und

für eine Einfachbindung oder für eine Doppelbindung steht.

21. Das Verfahren nach Anspruch 11 bis 18,

dadurch gekennzeichnet, daß

der Extrakt mit Brasilin und/oder Hämatoxylin angereichert wird.

22. Das Verfahren nach einem der Ansprüche 11 bis 21,

dadurch gekennzeichnet, daß

der -gegebenenfalls angereicherte -Extrakt zu einem oral applizierbaren Arzneimittel konfektioniert wird.

23. Verfahren nach Anspruch 22,

dadurch gekennzeichnet, daß

Kapseln mit einem Extraktgehalt von 100 bis 700 mg erzeugt werden.

24. Ein Arzneimittel, mit einer

-vorbeugenden und/oder heilenden Wirkung auf mikrozirkulatorische Störungen,

-antidiabetischen Wirkung,

-Blutzucker-senkenden Wirkung,

-die Plättchenaggregation hemmenden Wirkung,

-die Erythrozyten-Verformbarkeit steigernden Wirkung,

-normalisierenden Wirkung auf die lysosomalen Enzyme, und/oder mit einer

-den Prostaglandin-Stoffwechsel beeinflußenden, hier insbesondere selektiv

Thromboxan $A_2$ hemmenden Wirkung,

dadurch gekennzeichnet, daß

das Arzneimittel entsprechend dem Verfahren nach einem der Ansprüche 11 bis 23 erhältlich ist.

25. Verwendung eines aus Pflanzenmaterial der zur Gattung Caesalpinia gehörenden Pflanzen extrahierbaren Extraktes, einer oder mehrerer -(Komponente)n dieses Extraktes und/oder einer oder mehrerer in diesem Extrakt vorkommenden oder davon abgeleiteten, jedoch synthetisch hergestellten Substanz(en) zur Herstellung eines Arzneimittels mit

-einer vorbeugenden und/oder heilenden Wirkung auf mikrozirkulatorische Störungen,

-einer antidiabetischen Wirkung,

-einer Blutzucker-senkenden Wirkung,

-einer die Plättchenaggregation hemmenden Wirkung,

-einer die Blutviskosität senkenden Wirkung,

-einer die Erythrozyten-Verformbarkeit steigernden Wirkung,

- einer normalisierenden Wirkung auf die lysosomalen Enzyme, und/oder mit

- einer den Prostaglandin-Stoffwechsel beeinflußenden, hier insbesondere selektiv

Thromboxan $A_2$ hemmenden Wirkung.

26. Die Verwendung nach Anspruch 25,

wobei die wirksame Substanz ein Benz(b)indeno-(2.1-d)pyran-Derivat entsprechend nachstehender allgemeiner Strukturformel (I) ist,

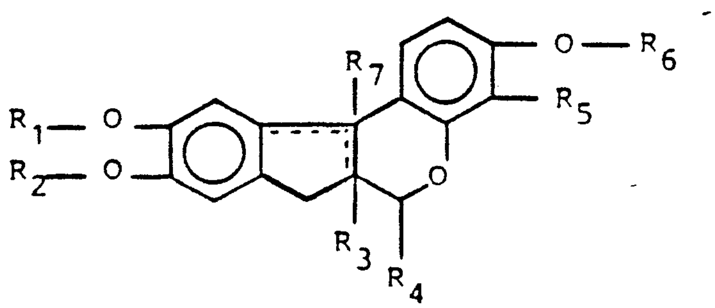

(I)

wobei

$R_1$, $R_2$ und $R_6$ gleich oder verschieden sein können und stehen für einen Wasserstoffrest, einen niederen Alkylrest, vorzugsweise Methylrest, einen Acetylrest, einen Benzylrest, oder $R_1$ und $R_2$ gemeinsam eine Methylengruppe bilden;

$R_3$ und $R_7$ gleich oder verschieden sein können und stehen für einen Wasserstoffrest, Hydroxylrest oder Acetylrest;

$R_4$ für einen Wasserstoffrest, einen Hydroxylrest, einen niederen Alkylrest, vorzugsweise Methylrest, einen Acetylrest oder einen Benzylrest steht; und

- für eine Einfachbindung oder für eine Doppelbindung steht.

27. Die Verwendung nach Anspruch 25,

wobei die wirksame Substanz ein Benz(b)indeno-(2.1-d)pyran-Derivat entsprechend nachstehender allgemeiner Strukturformel (II) ist,

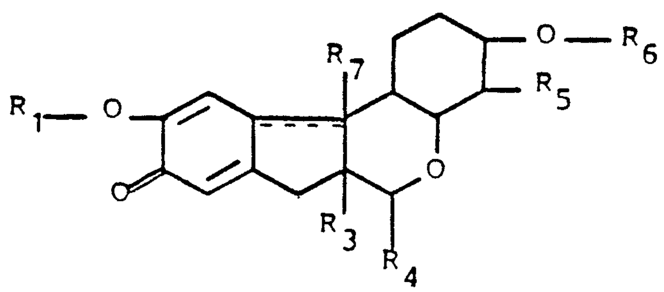

(II)

wobei

$R_1$ und $R_6$ gleich oder verschieden sein können und stehen für einen Wasserstoffrest, einen niederen Alkylrest, vorzugsweise Methylrest, einen Acetylrest, einen Benzylrest, oder $R_1$ und $R_6$ gemeinsam eine Methylengruppe bilden;

$R_3$ und $R_7$ gleich oder verschieden sein können und stehen für einen Wasserstoffrest, Hydroxylrest oder Acetylrest;

$R_4$ für einen Wasserstoffrest oder eine Oxogruppe steht;

$R_5$ für einen Wasserstoffrest, einen Hydroxylrest, einen niederen Alkylrest, vorzugsweise Methylrest, einen Acetylrest oder einen Benzylrest steht, und

- für eine Einfachbindung oder für eine Doppelbindung steht.

28. Die Verwendung nach Anspruch 25,

wobei die wirksame Substanz Brasilin und/oder Brasilein ist.

29. Die Verwendung nach Anspruch 25,

wobei die wirksame Substanz Hämatoxylin und/oder Hämatein ist.

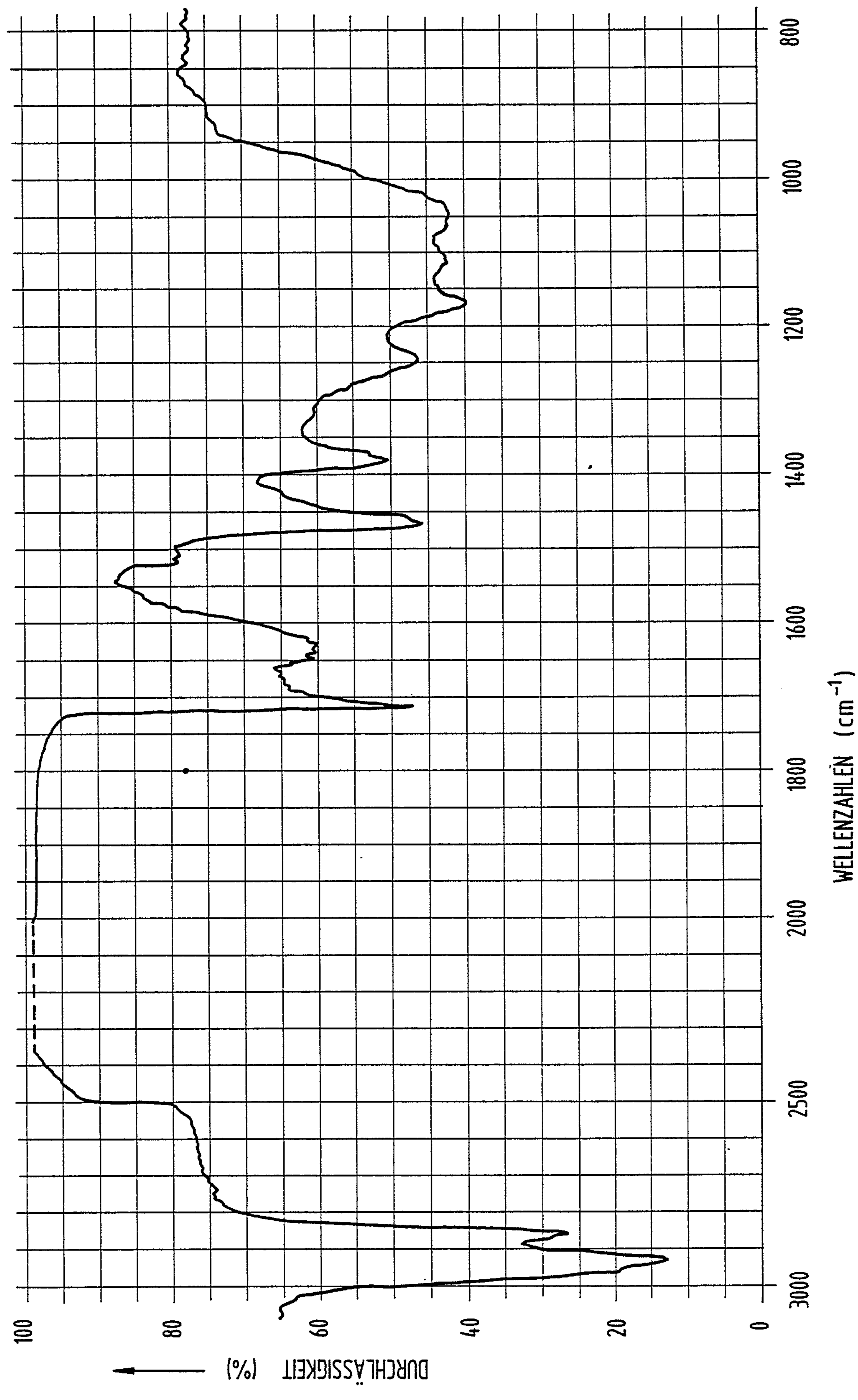

DURCHLÄSSIGKEIT (%)
100
80
60
40
20
0
WELLENZAHLEN (cm$^{-1}$)
3000
2500
2000
1800
1600
1400
1200
1000
800


FIG. 1

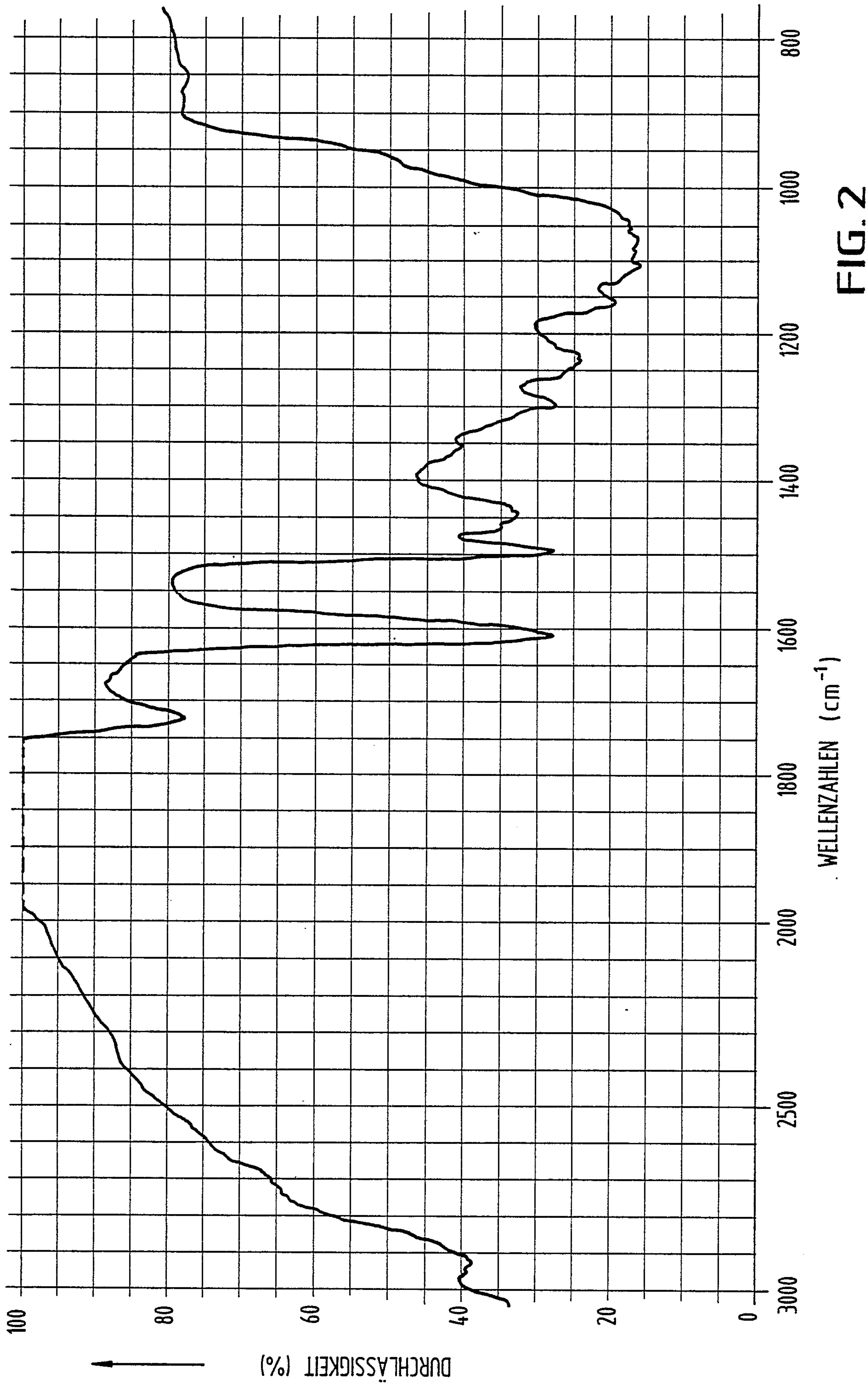

100
80
60
40
20
0
DURCHLÄSSIGKEIT (%)
3000
2500
2000
1800
1600
1400
1200
1000
800
WELLENZAHLEN (cm$^{-1}$)


FIG. 2

FIG. 3

GLUKOSETOLERANZTEST (GTT)
FALL: 3

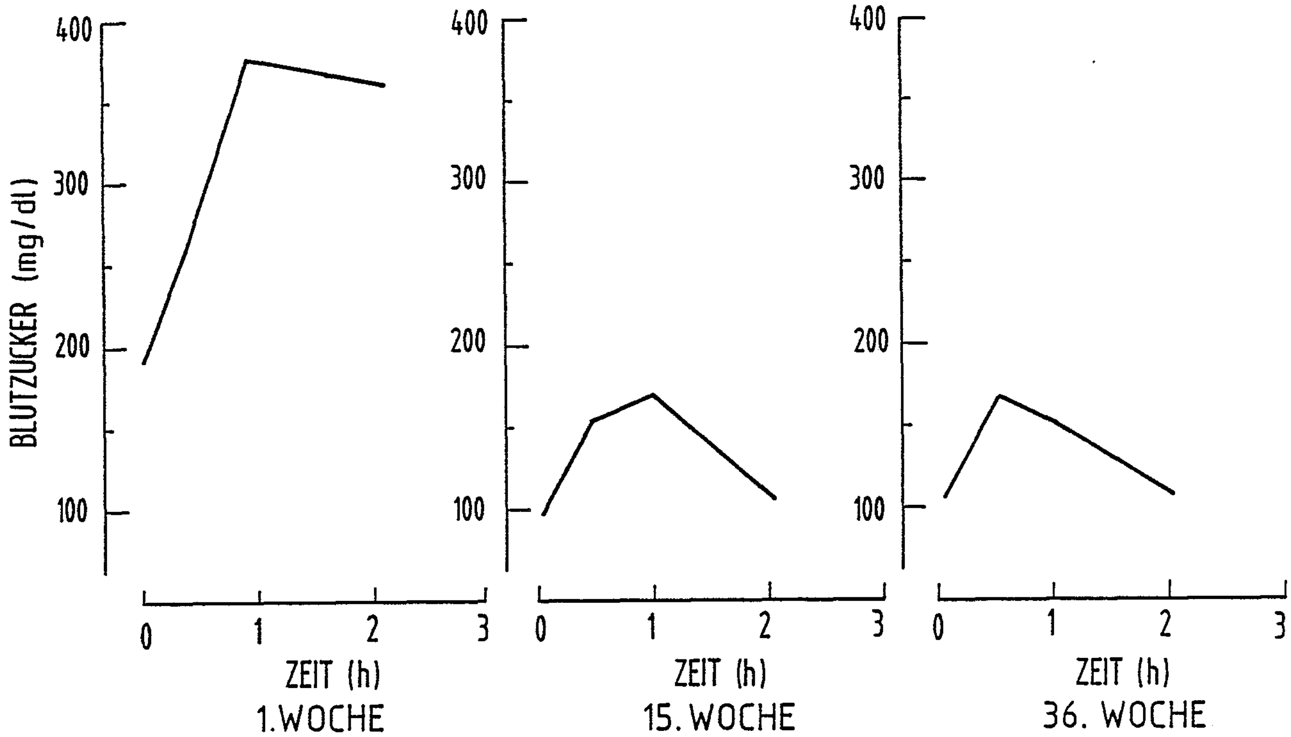


FIG. 4

GLUKOSETOLERANZTEST (GTT)
FALL: 6

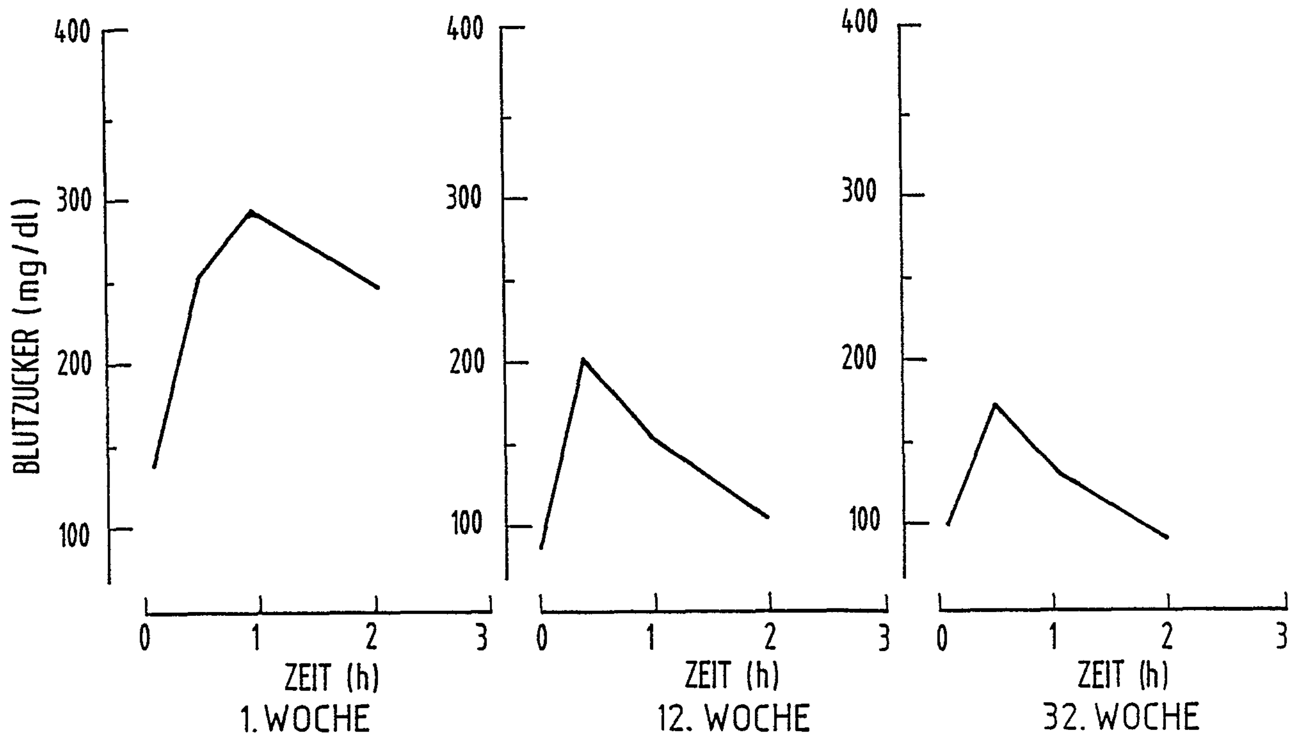

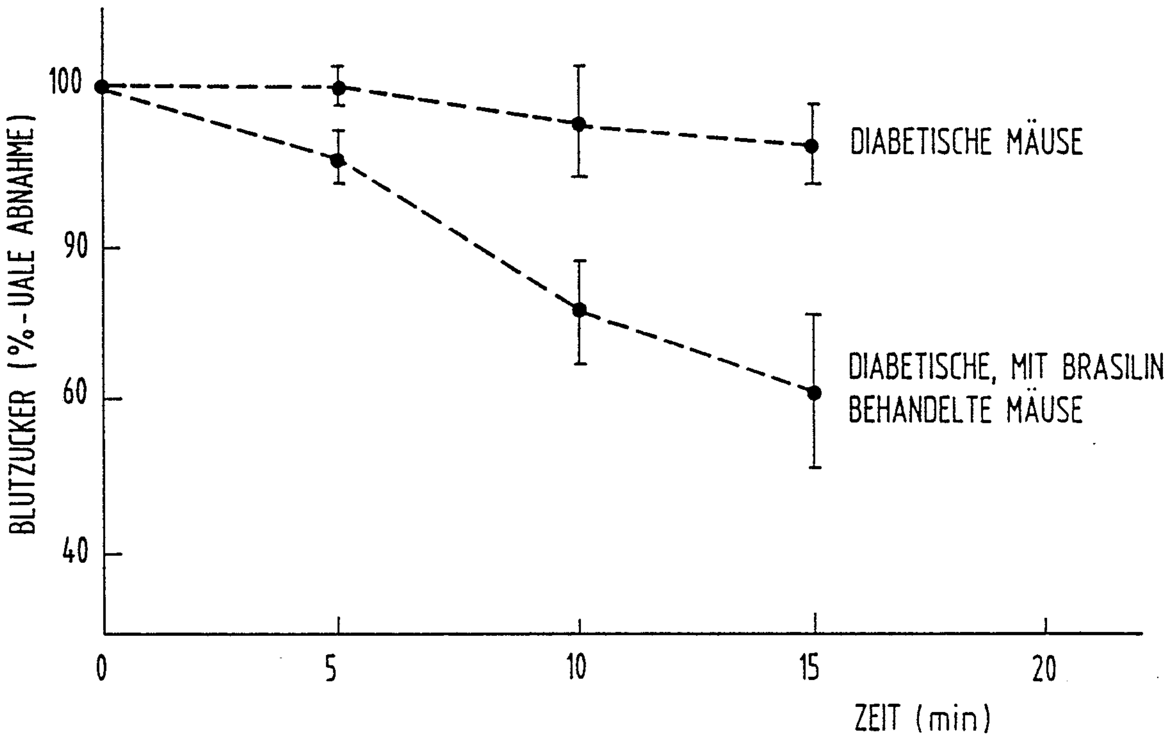
100
90
60
40
BLUTZUCKER (%-UALE ABNAHME)
DIABETISCHE MÄUSE
DIABETISCHE, MIT BRASILIN
BEHANDELTE MÄUSE
0
5
10
15
20
ZEIT (min)

FIG. 5

FIG. 6

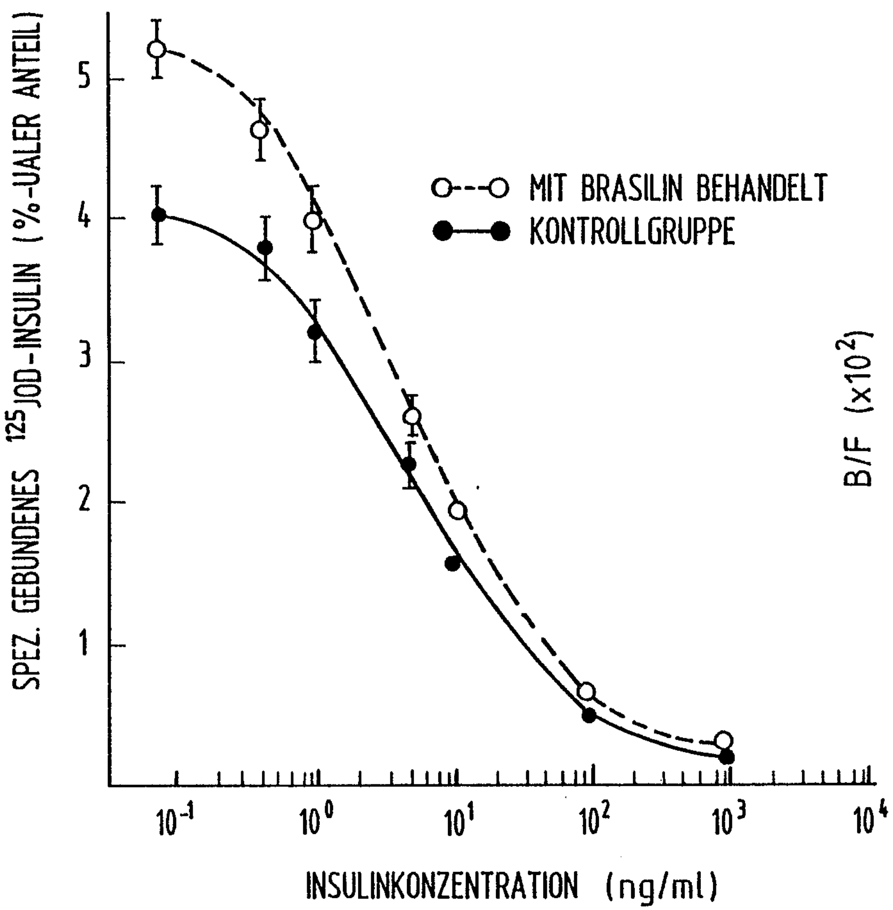
SPEZ. GEBUNDENES $^{125}$JOD-INSULIN (%-UALER ANTEIL)
5
4
3
2
1
MIT BRASILIN BEHANDELT
KONTROLLGRUPPE
$10^{-1}$
$10^{0}$
$10^{1}$
$10^{2}$
$10^{3}$
$10^{4}$
INSULINKONZENTRATION (ng/ml)

FIG. 7

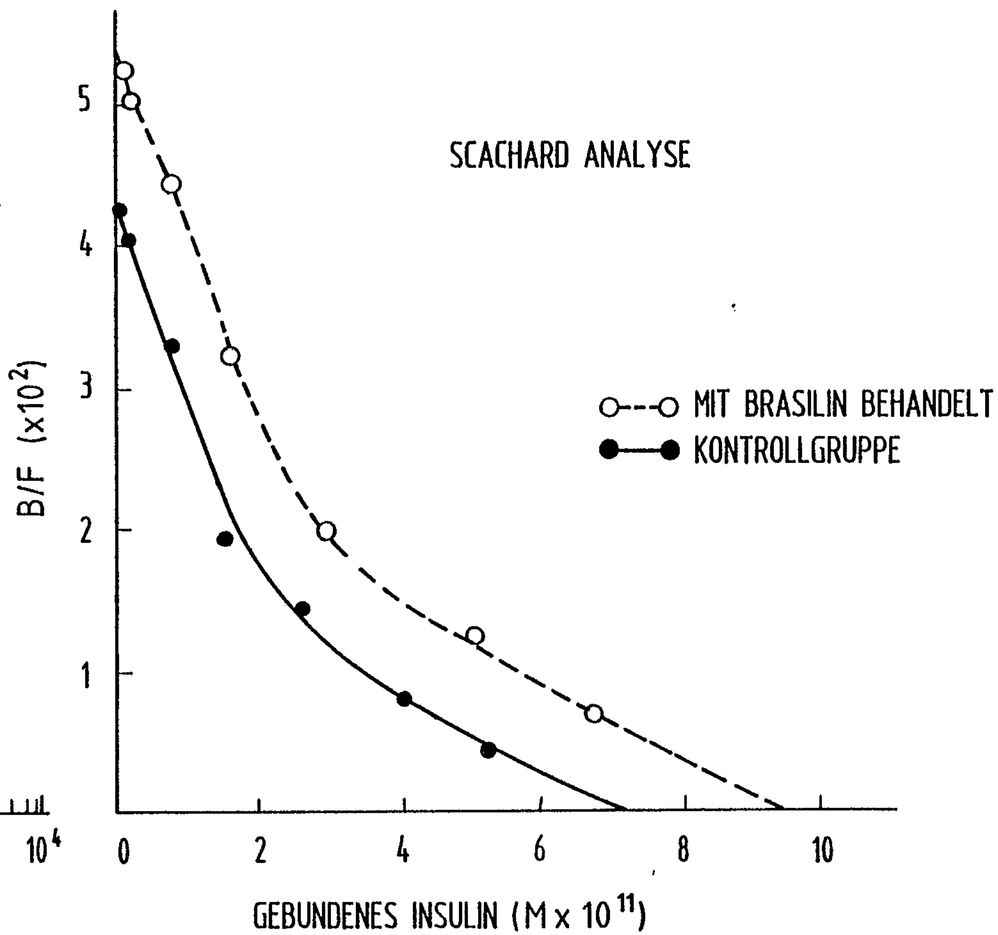
SCACHARD ANALYSE
B/F ($x10^2$)
5
4
3
2
1
MIT BRASILIN BEHANDELT
KONTROLLGRUPPE
0
2
4
6
8
10
GEBUNDENES INSULIN ($M \times 10^{11}$)